(19) Europäisches Patentamt
European Patent Office
Office européen des brevets

(11) **EP 3 603 505 A1**

(12) **EUROPEAN PATENT APPLICATION**
published in accordance with Art. 153(4) EPC

(43) Date of publication:
**05.02.2020 Bulletin 2020/06**

(51) Int Cl.:
***A61B 5/11*** *(2006.01)*

(21) Application number: **17902676.0**

(86) International application number:
**PCT/JP2017/012494**

(22) Date of filing: **27.03.2017**

(87) International publication number:
**WO 2018/179072 (04.10.2018 Gazette 2018/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Fujitsu Limited**
**Kawasaki-shi, Kanagawa 211-8588 (JP)**

(72) Inventors:
• **SASAMOTO, Yuki**
**Kawasaki-shi**
**Kanagawa 211-8588 (JP)**

• **INOMATA, Akihiro**
**Kawasaki-shi**
**Kanagawa 211-8588 (JP)**
• **HOTTA, Shinji**
**Kawasaki-shi**
**Kanagawa 211-8588 (JP)**

(74) Representative: **Hoffmann Eitle**
**Patent- und Rechtsanwälte PartmbB**
**Arabellastraße 30**
**81925 München (DE)**

(54) **INFORMATION PROCESSING SYSTEM, INFORMATION PROCESSING DEVICE, AND INFORMATION PROCESSING METHOD**

(57)     A section in which a motion for adjusting the posture of a living body or an object is identified accurately. An information processing device (101) extracts a main motion section (111) and an adjustment motion section candidate (112) from an acquiring sensor (102) as illustrated in (1) to (3) of FIG. 1. Next, the information processing device (101) calculates a relationship likelihood value that the adjustment motion section candidate (112) is an adjustment motion section for the main motion section (111) based on the feature amount of the main motion section (111) and the feature amount of the adjustment motion section candidate (112) as illustrated in (4) of FIG. 1. Then, the information processing device (101) determines whether the adjustment motion section candidate (112) is an adjustment motion section for the main motion section (111) based on the calculated relationship likelihood value as illustrated in (5) of FIG. 1.

FIG. 1

EP 3 603 505 A1

# EP 3 603 505 A1

**Description**

TECHNICAL FIELD

The present invention relates to an information processing system, an information processing device, and an information processing method.

BACKGROUND ART

Conventionally, a section in which a motion for adjusting the posture of a living body such as a patient is made with respect to a motion made by the living body may be identified, and a motion feature of the living body may be evaluated using the identified section.

There is a prior art for determining a type and strength of a physical motion by combining results obtained by performing an arithmetic operation predetermined for an acceleration on an output acceleration, and performing an arithmetic operation predetermined for an angular velocity on an output angular velocity, for example. The acceleration and the angular velocity are measured by a body-side device including a motion sensor capable of detecting an acceleration in one direction and a rotational angular velocity in one plane. There is also a technique for calculating the posture of a body, a movable range, a movement speed, or a smoothness of a movement according to change of a detected value detected by a sensor by using a detected value input from the sensor based on a relative position data as an initial set value. In addition, there is a technique for identifying an action pattern of a person carrying a device by comparing the combination of signals input by an acceleration sensor and a gyro with various action model patterns when the person carrying the device starts the action.

CITATION LIST

PATENT DOCUMENT

Patent Document 1: Japanese Laid-open Patent Publication No. 2002-78697
Patent Document 2: Japanese Laid-open Patent Publication No. 2010-273746
Patent Document 3: Japanese Laid-open Patent Publication No. 10-24026

SUMMARY OF INVENTION

TECHNICAL PROBLEM

However, according to the prior art, it may be difficult to identify the section in which a motion for adjusting the posture of the living body is made in some cases. For example, when a section in which a motion for adjusting the posture of a patient having a feature different from a healthy person is made, a section in which the motion for adjusting the posture of the living body may not be identified correctly.

In one aspect, an object of the present invention is to provide an information processing system, an information processing device, and an information processing method capable of accurately identifying a section in which a motion for adjusting the posture of a living body or an object is made.

SOLUTION TO PROBLEM

According to an aspect of the embodiments, an information processing system, an information processing device, and an information processing method perform, in the information processing system capable of communicating with a sensor configured to measure at least a motion among a state or the motion of a living body or an object, extracting, from time-series data obtained by the sensor, a first data section, in which the state of the living body or the object is changed to a predetermined state or the living body or the object makes a predetermined motion, extracting, from the time-series data, a second data section different from the first data section, calculate, based on a feature amount of the first data section and a feature amount of the second data section, a likelihood value that indicates likelihood that the second data section is an adjustment motion section during which a motion for adjusting a posture of the living body or the object is made for the first data section, and determining, based on the likelihood value that is calculated, whether the second data section is an adjustment motion section for the first data section.

ADVANTAGEOUS EFFECTS OF INVENTION

In one aspect, a section in which a motion for adjusting the posture of a living body or an object may be identified accurately.

BRIEF DESCRIPTION OF DRAWINGS

FIG. 1 is an explanatory diagram illustrating a motion example of an information processing system 100 according to the present embodiment.

FIG. 2 is an explanatory diagram illustrating a configuration example of the information processing system 100.

FIG. 3 is an explanatory diagram illustrating a configuration example of hardware of an information processing device 101 and a data acquisition device 201.

FIG. 4 is an explanatory diagram illustrating a functional configuration example of the information processing system 100.

FIG. 5 is a flowchart illustrating an example of a somatosensation evaluation processing procedure.

FIG. 6 is a flowchart illustrating an example of an adjustment motion section determination processing procedure.

FIG. 7 is an explanatory diagram illustrating an example of extraction of a main motion section.

FIG. 8 is an explanatory diagram illustrating an example of extraction of adjustment motion section candidates.

FIG. 9 is an explanatory diagram illustrating an example of extraction of model parameters of the main motion section corresponding to each adjustment motion section candidate.

FIG. 10 is an explanatory diagram illustrating an example of extraction of model parameters of each adjustment motion section candidate.

FIG. 11 is an explanatory diagram illustrating a calculation example of the relationship likelihood value of a smooth-normal model.

FIG. 12 is an explanatory diagram illustrating a calculation example of the relationship likelihood value of a smooth-abnormal model.

FIG. 13 is an explanatory diagram (No. 1) illustrating a calculation example of the relationship likelihood value of a non-smooth-normal model.

FIG. 14 is an explanatory diagram (No. 2) illustrating a calculation example of the relationship likelihood value of a non-smooth-normal model.

FIG. 15 is an explanatory diagram illustrating a calculation example of the relationship likelihood value of a non-smooth-abnormal model.

FIG. 16 is an explanatory diagram illustrating an example of a walking state of a patient uA in a first embodiment.

FIG. 17 is an explanatory diagram illustrating an example of sensor data during walking of the patient uA in the first embodiment.

FIG. 18 is an explanatory diagram illustrating an example of extraction of a main motion section in the first embodiment.

FIG. 19 is an explanatory diagram (No. 1) illustrating an example of extraction of an adjustment motion section candidate in the first embodiment.

FIG. 20 is an explanatory diagram (No. 2) illustrating an example of extraction of an adjustment motion section candidate in the first embodiment.

FIG. 21 is an explanatory diagram illustrating an example of extraction of model parameters of the main motion section corresponding to each adjustment motion section candidate in the first embodiment.

FIG. 22 is an explanatory diagram illustrating an example of extraction of model parameters of an adjustment motion section candidate in the first embodiment.

FIG. 23 is an explanatory diagram (No. 1) illustrating a calculation example of the relationship likelihood value of each motion model according to the first embodiment.

FIG. 24 is an explanatory diagram (No. 2) illustrating a calculation example of the relationship likelihood value of each motion model according to the first embodiment.

FIG. 25 is an explanatory diagram illustrating an example of determination of an adjustment motion section in the first embodiment.

FIG. 26 is an explanatory diagram illustrating an example of calculation of an evaluation value in the first embodiment.

FIG. 27 is an explanatory diagram illustrating an example of accumulation of evaluation values in the first embodiment.

DESCRIPTION OF EMBODIMENTS

Hereinafter, embodiments of a disclosed information processing system, information processing device, and information processing method will be described in detail with reference to the drawings.

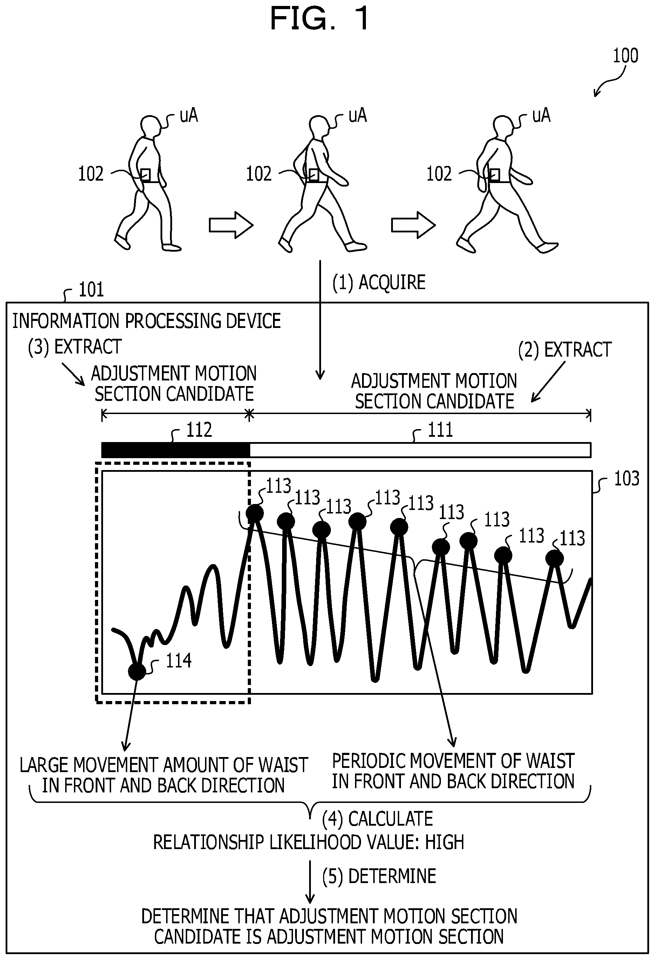

FIG. 1 is an explanatory diagram illustrating a motion example of an information processing system 100 according to the present embodiment. The information processing system 100 is a system that determines a section of a motion for adjusting the posture of a living body or an object in order to continuously evaluate the somatosensation of the living body or the object. Here, the living body is, for example, a person to be treated by a doctor, that is, a patient. Also, the object is, for example, a humanoid robot. In the description below, a living body is assumed to be a patient, and evaluation of the somatosensation of the patient is described. For example, the information processing system 100 is a system that provides a watching service for grasping the state of a patient, or provides a service for Personal Health Record (PHR).

The somatosensation is described below. The somatosensation is a generic term for skin sensation and deep sensation. The somatosensation is important in human movement control, and a specific disease may cause lack or decrease in somatosensation, and decrease in somatosensation may lead to danger such as falling. When there is an abnormality in somatosensation, posture adjustment becomes difficult. Thus, abnormal feature is easily found in transitioning from a middle of a motion or from one state/motion to another state/motion. Therefore, a motion feature in transition is considered to be important in the understanding and evaluation of human movement control and nervous system abnormalities. For example, a patient having an abnormality in the cranial nervous system such as a patient of concussion, Parkinson's disease, or multiple sclerosis is considered to have the feature of posture adjustment in motion transition different from that of a healthy person.

(Reference Document 1: Martina Mancini and 4 others, "ANTICIPATORY POSTURAL ADJUSTMENTS PRIOR TO STEP INITIATION ARE HYPOMETRIC IN UNTREATED PARKINSON'S DISEASE: AN ACCELEROMETER-BASED APPROACH", European Journal of Neurology, Vol. 16, pp. 1028-1034, 2009)
(Reference Document 2: Jebb G. Remelius and 3 others, "Gait Initiation in Multiple Sclerosis", Motor Control, Vol. 12, pp. 93-108, 2008)

Also, for example, in motion transition of a healthy person, a motion with emphasis on efficiency can be seen even though the posture is unstable. Here, a motion with emphasis on efficiency is, for example, a motion that increases a motion speed or a motion for smooth transition of motion. On the other hand, when there is an abnormality in somatosensation or the cranial nervous system, an early response is difficult to be made at the time of motion transition. Thus, the efficiency of the motion may be ignored and a motion for maintaining the stability of the posture as much as possible may appear, for example. In addition, for performing exercise such as walking or keeping a standing position, posture adjustment is performed while recognizing the state and position of hands and feet. Thus, when there is lack or decrease in somatosensation, or there is an abnormality in somatosensation, fine posture adjustment is not possible, and as a result, disturbance of motion is easily caused in the exercise.

Such posture adjustment is also found in daily life. Thus, if a section during which a posture adjustment motion is made can be automatically detected to quantify the above-described motion, it is considered that abnormality in the somatosensation of a patient or other condition may be evaluated continuously in daily life. Hereinafter, the section during which a motion for adjusting the posture is made may be referred to as an "adjustment motion section".

Therefore, there is a technique of detecting an adjustment motion section using a relatively inexpensive wearable sensor. For example, a possible technique for detecting an adjustment motion section is to perform threshold value processing on a waist acceleration sensor.

(Reference Document 3: Rigoberto Martinez-Mendez and two others, "Detection of anticipatory postural adjustments prior to gait initiation using inertial wearable sensors", Journal of NeuroEngineering and Rehabilitation, Vol. 8, No. 17, 2011)

However, by the above-described technique, it is difficult to detect an adjustment motion section of a patient with high accuracy when a motion feature of the posture adjustment is different from an assumed motion feature, which is a motion feature of a healthy person, for example. In order to detect an adjustment motion section accurately, for example, tuning for each patient is performed. The tuning may be changing a threshold value or changing a waist acceleration waveform, for example. Thus, in the case where there are many patients, such tuning for individual patient is difficult.

Another possible technique is to detect a posture adjustment section by using a section of the first step that is identified by a gyro sensor on a foot as a clue.

(Reference Document 4: Martina Mancini and four others, "Validity and reliability of an IMU-based method to detect APAs prior to gait initiation", Gait & Posture, Vol. 43, pp. 125-131, 2016)

However, by the above-described technique, it is difficult to detect an adjustment motion section of a patient with high accuracy when a motion feature of the posture adjustment or a motion feature of the first step is different from an assumed motion feature, which is a motion feature of a healthy person, for example. In order to detect an adjustment motion section accurately, for example, tuning for each patient is performed. Thus, in the case where there are many patients, such tuning for individual patient is difficult.

Still another possible technique is to detect a posture adjustment by learning data of an inertial sensor and a foot pressure sensor at the start of walking in advance, and using a rule based on the learned data.
(Reference Document 5: Domen Novak and nine others, "Automated detection of gait initiation and termination using wearable sensors", Medical Engineering & Physics, Vol. 25, pp. 1713-1720, 2013)

However, in the above-described technique, additional learning may occur when, for example, the motion at the time of posture adjustment changes with the lapse of time due to a state change such as recovery or worsening.

In addition, as a motion feature different from that of a healthy person, a concussion patient is characterized in that the movement amounts of the center of gravity and foot center until the first step is smaller than those of a healthy person. Alternatively, a Parkinson's disease patient makes abnormal start of walking, which is called Freezing of Gait, and when an abnormal start of walking is made, an abnormal posture adjustment motion appears.

In addition, when the situations at the time of making a motion are different, features of posture adjustment may be different. The different situations may be generated when, for example, walking is started in a soft floor, a patient is in a hurry, a patient has a leg injury, and the like. In such cases, simply extracting the section of posture adjustment, converting it into a feature amount, and comparing and evaluating the feature amount does not tell that a situation at the time of making a motion is different, and may lead to evaluation that the feature of the posture adjustment is performed by the patient or that the somatosensation is abnormal.

Therefore, the present embodiment utilizes a fact that a healthy person or a patient performs balance control to stabilize a motion in an adjustment motion section, and the feature of this balance control is affected by a motion before or after the adjustment motion section. More specifically, the present embodiment extracts a main motion section and a candidate for an adjustment motion section from measurement data of a motion of a patient, calculates a likelihood value from the feature amounts of the main motion section and the candidate for an adjustment motion section, and determines whether the candidate for the adjustment motion section is an adjustment motion section based on the calculated likelihood value.

Here, the main motion is, for example, a motion accompanied by a motion such as turning, stair climbing, and change of posture. In addition, the main motion may include a static state. The adjustment motion is a motion of posture adjustment during the main motion or during transition from one main motion to another main motion. The adjustment motion is, for example, a motion of the trunk when walking is started from a static state. Also, the likelihood value described above is a value indicating the likelihood that the candidate of the adjustment motion section is the adjustment motion section for the main motion section. Then, it can be said that the above-described likelihood value indicates the strength of the relationship between the candidate of the adjustment motion section and the main motion section. Hereinafter, the above-described likelihood value may be referred to as "relationship likelihood value". Further, the candidate of the adjustment motion section may be referred to as "adjustment motion section candidate".

A motion example of the information processing system 100 will be described with reference to FIG. 1. The information processing system 100 includes an information processing device 101 and a sensor 102. The information processing device 101 and the sensor 102 are connected to be communicable. The information processing device 101 is, for example, a server. The sensor 102 measures at least a motion among a state and a motion of a user uA. The sensor 102 is attached to, for example, the waist of the user uA. The user uA is a patient to be treated. FIG. 1 illustrates an example where the user uA starts walking.

The information processing device 101 acquires time-series data from the sensor 102 as illustrated in (1) of FIG. 1. A graph 103 in FIG. 1 illustrates acquired time-series data. The graph 103 illustrates the acceleration of the waist in the front and back direction. Hereinafter, data acquired from the sensor 102 may be referred to as "sensor data".

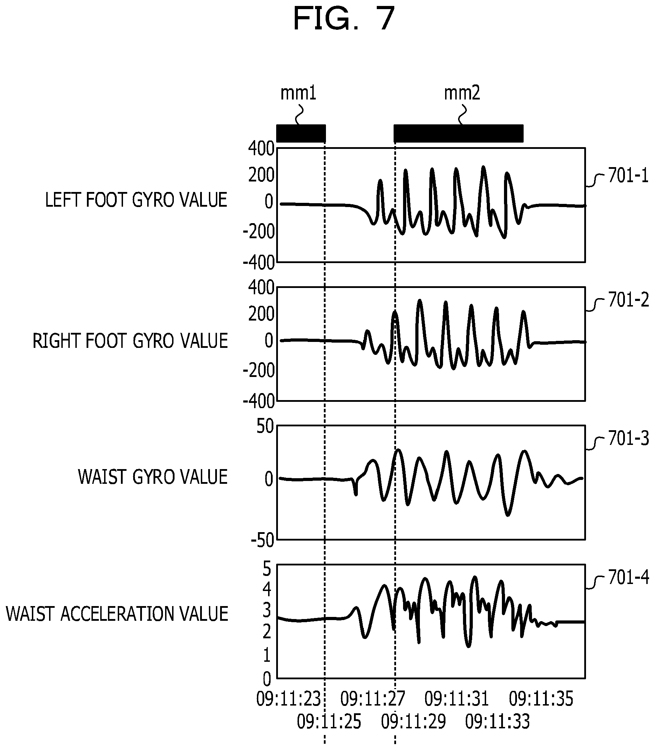

Next, as illustrated in (2) of FIG. 1, the information processing device 101 extracts, from the sensor data, a main motion section as a first data section in which the state of the patient is changed to a predetermined state or the patient makes a predetermined motion. The predetermined state is, for example, a static state. The predetermined motion is, for example, walking, turning, standing up, or the like. A more specific method of extracting the main motion section is illustrated in FIG. 7 and other drawings. In the example of FIG. 1, the information processing device 101 extracts a main motion section 111. In addition, in FIG. 1, the main motion section 111 is illustrated as a white rectangle.

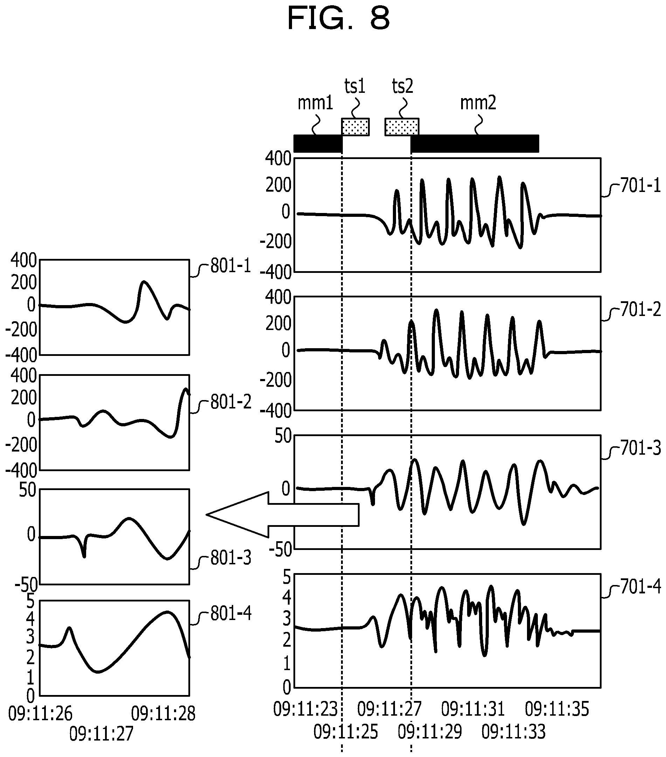

Then, as illustrated in (3) of FIG. 1, the information processing device 101 extracts, from the sensor data, an adjustment motion section candidate as a second data section different from the main motion section. A more specific method of extracting the adjustment motion section candidate is illustrated in FIG. 8 and the like. In the example of FIG. 1, the information processing device 101 extracts an adjustment motion section candidate 112. In FIG. 1, the adjustment motion section candidate 112 is illustrated as a black rectangle.

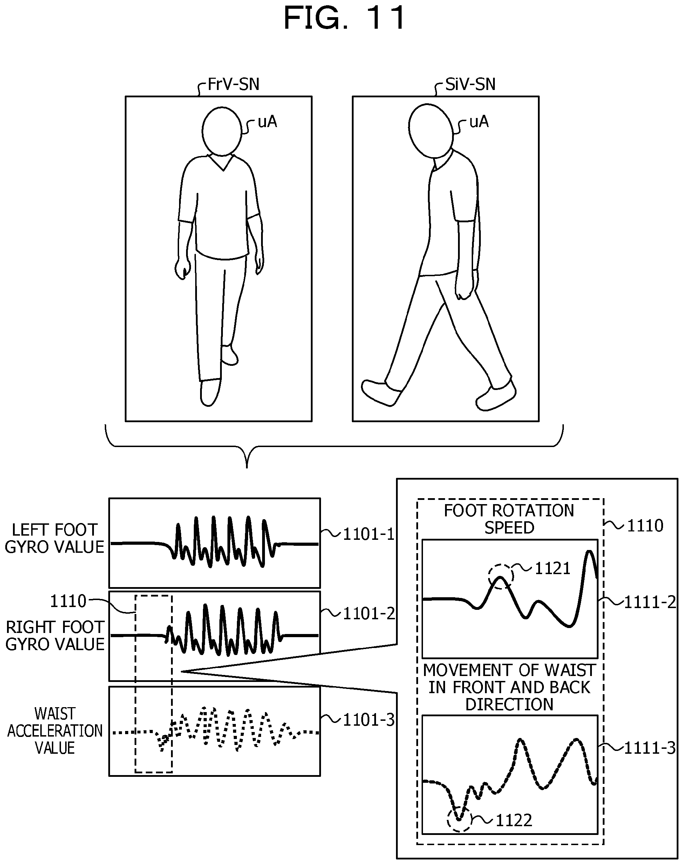

Next, the information processing device 101, as illustrated in (4) of FIG. 1, calculates a relationship likelihood value that the adjustment motion section candidate 112 is an adjustment motion section for the main motion section 111 based on the feature amount of the main motion section 111 and the feature amount of the adjustment motion section candidate 112. Examples of calculation of the relationship likelihood value are illustrated in FIGS. 11 to 15. The relationship likelihood value to be calculated may be represented by a numerical value, or may be represented by characters such as "high", "medium", and "low".

In the example of FIG. 1, the feature amount of the main motion section 111 is, for example, information on black circles 113 in the main motion section 111, and the black circles 113 indicate peak values of the waist in the front and back direction. The peak values are local minimum values or the local maximum values. Therefore, the feature amount of the main motion section 111 is, for example, the acceleration values at the black circles 113, the time section of the black circles 113, and the number of the black circles 113 as information on the black circles 113. In addition, in the example of FIG. 1, the black circles 113 indicate that there is a periodic movement of the waist in the front and back direction. The periodic movement of the waist in the front and back direction indicates that the walking motion is normal.

Further, the feature amount of the adjustment motion section candidate 112 is, for example, information on a black circle 114 in the adjustment motion section candidate 112, and the black circle 114 indicates a peak value of the waist in the front and back direction. Therefore, the feature amount of the adjustment motion section candidate 112 is, for example, an acceleration value at the black circle 114. And in the example of FIG. 1, the black circle 114 indicates that there is a large movement amount of the waist in the front and back direction. A large movement amount of the waist in the front and back direction indicates a motion up to making the first step, and indicates that the motion is a smooth adjustment motion for making a walking motion.

As described above, in the example of FIG. 1, after a smooth adjustment motion for a waking motion is made, a normal walking motion is made. Thus, it can be said that the feature of the adjustment motion section candidate 112 is affected by the main motion section 111. Therefore, the information processing device 101 calculates the relationship likelihood value that the adjustment motion section candidate 112 is the adjustment motion section for the main motion section 111 as "high".

Next, as illustrated in (5) of FIG. 1, the information processing device 101 determines whether the adjustment motion section candidate 112 is an adjustment motion section for the main motion section 111 based on the calculated relationship likelihood value. In the example of FIG. 1, the calculated relationship likelihood value is "high". Thus, the information processing device 101 determines that the adjustment motion section candidate 112 is the adjustment motion section for the main motion section 111.

As described above, the information processing device 101 determines whether the adjustment motion section candidate 112 is the adjustment motion section for the main motion section 111 using the relationship between the main motion section 111 and the adjustment motion section candidate 112. Therefore, the adjustment motion section can be determined accurately. Furthermore, the information processing device 101 can categorize the adjustment motion section by extracting the relationship between the main motion section 111 and the adjustment motion section candidate 112, thereby enabling comparison and evaluation.

In the example of FIG. 1, the main motion section 111 follows the adjustment motion section candidate 112, but the adjustment motion section candidate 112 may follow the main motion section 111. In addition, the main motion section 111 may be divided into a plurality pieces. For example, in the example of FIG. 1, the information processing device 101 may determine whether the adjustment motion section candidate 112 is the adjustment motion section based on the relationship between three or more sections such as the adjustment motion section candidate 112, the top portion of the main motion section 111, and the remaining portion of the main motion section 111.

Further, the relationship between the main motion and the adjustment motion is not limited to the combination in which the adjustment motion appearing in FIG. 1 is smooth and the main motion is normal. For example, the information processing device 101 may determine whether the adjustment motion section candidate 112 is an adjustment motion section using a motion model obtained by combining the features of the main motion and the adjustment motion. The motion model indicates a combination of whether the main motion is normal and whether the adjustment motion is smooth. Further, the information processing device 101 may determine whether the adjustment motion section candidate 112 is an adjustment motion section using a plurality of motion models. Then, the plurality of motion models is different from each other in whether the main motion is normal, whether the adjustment motion is smooth, the feature amount of the main motion or adjustment motion, or the value of the feature amount of the main motion or adjustment motion, or any combination thereof.

Specifically, the plurality of motion models includes a smooth-normal model, a smooth-abnormal model, a non-smooth-normal model, and a non-smooth-abnormal model. The smooth-normal model is a combination of a smooth adjustment motion and a normal main motion. The smooth-abnormal model is a combination of a smooth adjustment motion and an abnormal main motion. The non-smooth-normal model is a combination of a non-smooth adjustment motion and a normal main motion. The non-smooth-abnormal model is a combination of a non-smooth adjustment motion and an abnormal main motion. The details of the four motion models will be described below.

The smooth-normal model may be a motion model having a smooth posture adjustment and a normal motion feature before and after the posture adjustment that appear on a healthy person as described above. The smooth-normal model is, for example, a model having a motion feature of an adjustment motion for moving the waist in the front and back direction by a certain distance or longer at a certain speed or higher at the start of walking, and a motion of making the first step at a certain speed or higher after the adjustment motion.

The smooth-abnormal model may be a motion model having a smooth motion appearing at the posture adjustment stage, and an abnormal main motion after the posture adjustment. The smooth-abnormal model is, for example, a model having a motion feature of a quick waist motion appearing at the first step, and a false step, a totter, or the like at the time of walking after the first step.

The non-smooth-normal model may be a motion model in the case of making a motion for stabilizing the trunk at the time of adjustment motion so as not to lose the balance as much as possible even though balance control is difficult to be performed well due to a concussion or the like. The non-smooth-normal model is, for example, a model having a motion feature of a small movement in the front and back direction at the first step, and having a movement of the center of gravity to the fulcrum instead.

The non-smoothness-abnormal model may be a motion model having a feature of an adjustment motion and a main motion that appear on a patient of a cranial nervous system disease such as Parkinson's disease such as Freezing of Gait at the start of walking, for example. Here, when a patient with Parkinson's disease makes Freezing of Gait, it is difficult for the patient to make the first step well, and thus the patient tries to keep the balance. Therefore, the non-smooth-abnormal model is, for example, a model having a motion feature of the adjustment motion in which the center of gravity vibrates and the main motion of making short steps after the adjustment motion.

In addition, as each of the smooth-normal model, the smooth-abnormal model, the non-smooth-normal model, and the non-smooth-abnormal model, there may be a plurality of models having feature amounts of the main motion or adjustment motion that are different from each other, or values of a feature amount of the main motion or adjustment motion that are different from each other.

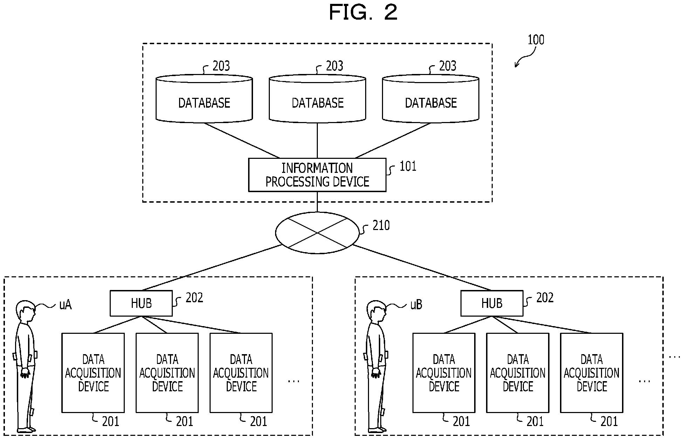

Further, in the example of FIG. 1, a living body is illustrated as a patient, but it may be a so-called diseased animal such as a dog, a cat, a horse or the like. Similarly, the object may be, for example, a robot imitating a dog, a cat, a horse or the like. Next, a configuration example of the information processing system 100 will be described with reference to FIG. 2.

(Configuration Example of Information Processing System 100)

FIG. 2 is an explanatory diagram illustrating a configuration example of the information processing system 100. The information processing system 100 includes the information processing device 101, data acquisition devices 201, hubs 202, and databases 203. The information processing device 101 and the data acquisition devices 201 are connected by a network 210 such as the Internet, a local area network (LAN), a wide area network (WAN), and the like, and the hubs 202.

The data acquisition devices 201 are devices which are attached to portions of a user and, each of which measures the movements of the corresponding portion. Then, each of the data acquisition devices 201 has the sensor 102, and measures the acceleration in three axes and the angular velocity in three axes of the portion to which the data acquisition device 201 is attached. In the example of FIG. 2, the plurality of data acquisition devices 201 is attached to portions of the user uA and a user uB.

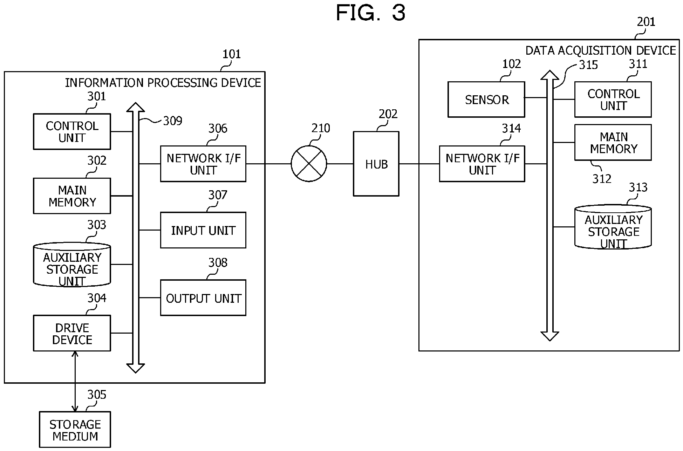

The databases 203 store data acquired from the data acquisition devices 201, data obtained by converting the acquired data, and feature amounts extracted from the data obtained by the conversion. Next, the hardware configuration of the information processing device 101 will be described with reference to FIG. 3.

(Hardware of Information Processing Device 101 and Data Acquisition Device 201)

FIG. 3 is an explanatory diagram illustrating a configuration example of hardware of the information processing device 101 and the data acquisition device 201. In FIG. 3, the information processing device 101 includes a control unit 301, a main storage unit 302, an auxiliary storage unit 303, a drive device 304, a network interface (I/F) unit 306, an input unit 307, and an output unit 308. The control unit 301 to the drive device 304, and the network I/F unit 306 to the output unit 308 are connected to each other by a bus 309.

The control unit 301 is an arithmetic processing device that controls the entire information processing device 101. For example, the control unit 301 is a Central Processing Unit (CPU). The main storage unit 302 is a volatile memory used as a work area of the control unit 301. Examples of the volatile memory include a dynamic random access memory (DRAM) and a static random access memory (SRAM).

The auxiliary storage unit 303 is a storage device that assists the main storage unit 302. For example, the auxiliary storage unit 303 is a Hard Disk Drive (HDD) or a Solid State Drive (SSD).

The drive device 304 is a control device that controls read/ write data from/to a storage medium 305 according to the control of the control unit 301. For example, if the storage medium 305 is an optical disk, the drive device 304 is an optical disk drive. If the storage medium 305 is a magnetic disk, the drive device 304 is a magnetic disk drive. The storage medium 305 is a portable storage medium. Further, the information processing device 101 may incorporate a semiconductor memory formed from a semiconductor element, and read/write data from/to a Universal Serial Bus (USB) flash drive including a drive device. The storage medium 305 may be detachable from the information processing device 101.

The network I/F unit 306 is connected to the network 210 through a communication line, and is connected to other devices via the network 210. The network I/F unit 306 controls the interface between the network 210 and the inside of the information processing device 101 to control input/output of data to/from external devices. For example, a modem or a LAN adapter can be used as the network I/F unit 306.

The input unit 307 is an input device that inputs information to the information processing device 101. For example, the input unit 307 is a mouse, a keyboard, or the like. The mouse is a device for moving a cursor, selecting a range, moving a window, changing a size, and the like. The keyboard is a device that has keys for inputting characters, numbers, various instructions, and the like to input data.

The output unit 308 is an input device that outputs information from the information processing device 101. For example, the output unit 308 is a display or the like. The display is a device for displaying data such as a document, an image, function information, and the like, as well as a cursor, an icon or a tool box. As the display, for example, a Cathode Ray Tube (CRT), a Thin Film Transistor (TFT) liquid crystal display, a plasma display or the like may be used.

The data acquisition device 201 includes a control unit 311, a main storage unit 312, an auxiliary storage unit 313, the sensor 102, and a network I/F unit 314. The sensor 102 and the control unit 311 to the network I/F unit 314 are connected to each other by a bus 315.

The control unit 311 is an arithmetic processing unit that controls the entire data acquisition device 201. For example, the control unit 311 is a CPU. The main storage unit 312 is a volatile memory used as a work area of the control unit 311. The volatile memory is, for example, a DRAM, an SRAM or the like.

The auxiliary storage unit 313 is a storage device that assists the main storage unit 312. For example, the auxiliary storage unit 313 is an SSD or the like.

The network I/F unit 314 is connected to the hub 202 by wireless communication or wired communication, and is connected from the hub 202 to other devices via the network 210. For example, a short range wireless communication interface or the like can be used as the network I/F unit 314.

(Functional Configuration Example of Information Processing System 100)

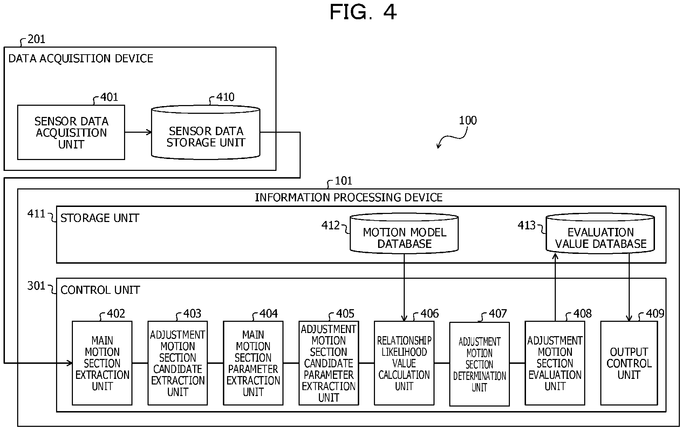

FIG. 4 is an explanatory diagram illustrating a functional configuration example of the information processing system 100. The data acquisition device 201 includes a sensor data acquisition unit 400. The control unit 301 includes a main motion section extraction unit 402, an adjustment motion section candidate extraction unit 403, a main motion section parameter extraction unit 404, an adjustment motion section candidate parameter extraction unit 405, and a relationship likelihood value calculation unit 406, and an adjustment motion section determination unit 407. Furthermore, the control unit 301 includes an adjustment motion section evaluation unit 408 and an output control unit 409. The control unit 301 implements the function of each unit by executing programs stored in the main storage unit 302 and the auxiliary storage unit 303. The processing result of each unit is stored in the main storage unit 302 or the like.

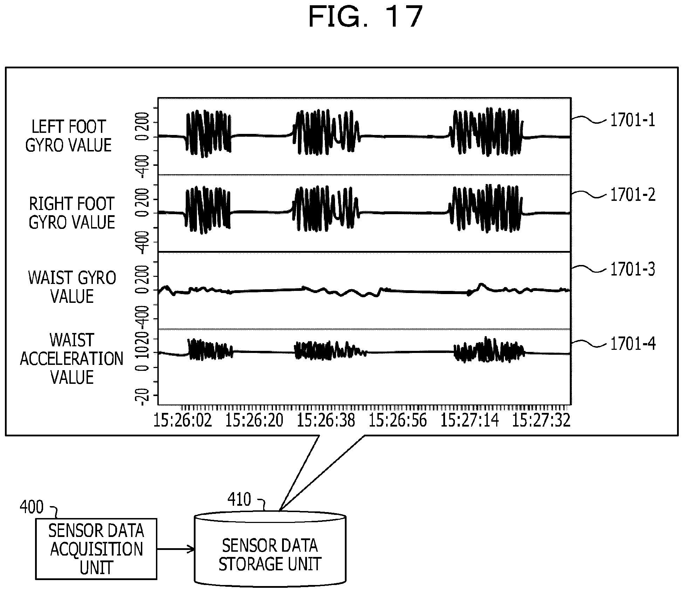

The data acquisition device 201 can also access a sensor data storage unit 410. The sensor data storage unit 410 is stored in an storage area of the main storage unit 312, the auxiliary storage unit 313, or the like. An example of the stored content of the sensor data storage unit 410 is illustrated in FIG. 17.

In addition, the information processing device 101 can access a storage unit 411. The storage unit 411 includes a motion model database 412 and an evaluation value database 413.

A sensor data acquisition unit 401 acquires sensor data measured by the sensor 102 and accumulates the sensor data in the sensor data storage unit 410. Thereby, the sensor data acquisition unit 401 can provide the accumulated sensor data to the main motion section extraction unit 402.

The main motion section extraction unit 402 extracts a main motion section from the sensor data. A more specific method of extracting the main motion section by the main motion section extraction unit 402 is illustrated in FIG. 7 and the like. As a result, the main motion section extraction unit 402 can provide the main motion section to be processed by the main motion section parameter extraction unit 404.

The adjustment motion section candidate extraction unit 403 extracts adjustment motion section candidates from the sensor data. A more specific method of extracting the adjustment motion section candidates by the adjustment motion section candidate extraction unit 403 is illustrated in FIG. 8 and the like. Accordingly, the adjustment motion section candidate extraction unit 403 can provide adjustment motion section candidates to be processed by the adjustment motion section candidate parameter extraction unit 405.

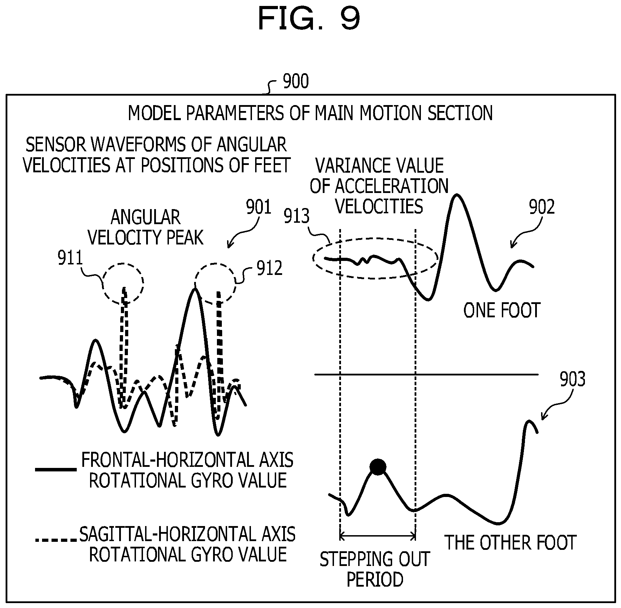

The main motion section parameter extraction unit 404 extracts the feature amount of the main motion section from the main motion section extracted by the main motion section extraction unit 402. Hereinafter, the feature amount of the main motion section may be referred to as "model parameter of the main motion section". An example of extraction of a model parameter of the main motion section by the main motion section parameter extraction unit 404 is illustrated in FIG. 9, and the like. Thereby, the main motion section parameter extraction unit 404 can provide a model parameter that the relationship likelihood value calculation unit 406 uses as an argument.

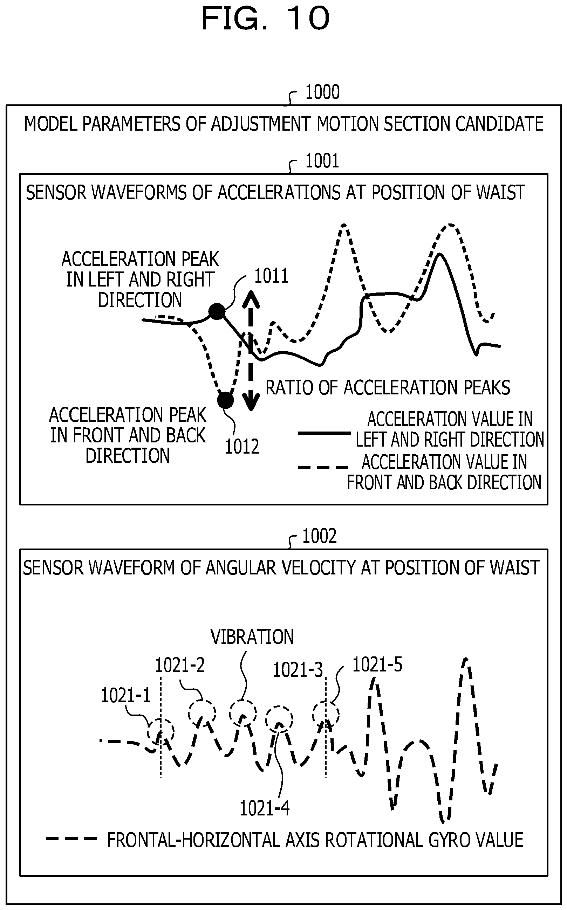

The adjustment motion section candidate parameter extraction unit 405 extracts the feature amount of the adjustment motion section candidate extracted by the adjustment motion section candidate extraction unit 403. Hereinafter, the feature amount of the adjustment motion section candidate may be referred to as "model parameters of the adjustment motion section candidate". An example of extraction of a model parameter of the adjustment motion section candidate by the adjustment motion section candidate parameter extraction unit 405 is illustrated in FIG. 10 and the like. Thereby, the adjustment motion section candidate parameter extraction unit 405 can provide a model parameter that the relationship likelihood value calculation unit 406 uses as an argument.

Based on the model parameter of the main motion section extracted by the main motion section parameter extraction unit 404 and the model parameter of the adjustment motion section candidate extracted by the adjustment motion section candidate parameter extraction unit 405, the relationship likelihood value calculation unit 406 calculates the relationship likelihood value of the above-described adjustment motion section candidate. Thereby, the relationship likelihood value calculation unit 406 can provide the relationship likelihood value that the adjustment motion section determination unit 407 uses as an argument.

Further, there may be a case where the relationship likelihood value calculation unit 406 calculates a first relationship likelihood value of the adjustment motion section candidate based on the model parameter of the main motion section, and a second relationship likelihood value of the adjustment motion section candidate based on the model parameter of the adjustment motion section candidate. In this case, the adjustment motion section determination unit 407 determines that the adjustment motion section candidate is the adjustment motion section for the main motion section if the first relationship likelihood value is a first threshold value or more and the second relationship likelihood value is a second threshold value or more, for example.

The adjustment motion section determination unit 407 determines whether the adjustment motion section candidate is an adjustment motion section for the main motion section based on the relationship likelihood value calculated by the relationship likelihood value calculation unit 406. Thereby, the adjustment motion section determination unit 407 can provide the adjustment motion section to a doctor.

The adjustment motion section evaluation unit 408 calculates an evaluation value to be used for evaluation of the somatosensation of the patient based on the determination result as to whether the adjustment motion section is the adjustment motion section for the main motion section and the feature amount representing the adjustment motion section. The feature amount representing the adjustment motion section includes the relationship likelihood value calculated by the relationship likelihood value calculation unit 406. For example, if the adjustment motion section determination unit 407 determines that the adjustment motion section is the adjustment motion section for the main motion section, the adjustment motion section evaluation unit 408 calculates, as the evaluation value of the somatosensation of the patient, the relationship likelihood value that has been calculated by the relationship likelihood value calculation unit 406 as it is. The calculated evaluation value is stored in the evaluation value database 413. Thereby, the adjustment motion section evaluation unit 408 can provide the evaluation value of the somatosensation to a doctor or the like.

The output control unit 409 outputs the evaluation value stored in the evaluation value database 413. The output control unit 409 may output the relationship likelihood value as a likelihood value. The output form is, for example, transmission to an external device by the output unit 308, or storage in a storage area such as the auxiliary storage unit 303 or the storage medium 305. Further, a doctor can view the output result by the output of the output unit 308, and compare and evaluate the somatosensation of the patient.

Here, as described with reference to FIG. 1, the relationship likelihood value calculation unit 406 may calculate the relationship likelihood value using a motion model. Specifically, the relationship likelihood value calculation unit 406 calculates the likelihood value of the adjustment motion section candidate described above based on a motion model, a model parameter of the main motion section, and a model parameter of the adjustment motion section candidate. In addition, the relationship likelihood value calculation unit 406 may calculate the likelihood value of the adjustment motion section candidate described above corresponding to each of a plurality of motion models based on corresponding one of the motion models described above, model parameters of the main motion sections, and model parameters of the adjustment motion section candidates.

Here, a motion model may be a model having a model parameter related to the movement of the waist or a model parameter related to the movement of the foot to be described below, or any combination thereof. The model parameter related to the movement of the waist is the amount, the speed, or the occurrence timing of movement in the left and right direction and/or the front and back direction, and/or rotation of the waist, or any combination thereof and/or a motion amount of the waist, a strength of stepping, or vibration, or any combination thereof. For example, a model parameter related to the motion of the waist may be a movement amount of the waist in the front and back direction and/or the left and right direction, the ratio between the movement amount of the waist in the front and back direction and the movement amount in the left and right direction, the rotational speed of the waist in the front and back direction and/or the left and right direction, or an amount indicating the vibration of the motion of the waist.

The model parameter related to the movement of the foot is the amount, the speed, or the occurrence timing of movement and/or rotation of the foot in left and right direction and/or front and back direction, or any combination thereof and/or a motion amount, a strength of stepping, or vibration of the foot, or any combination thereof. For example, the model parameters related to the movement of the foot may be the rotational speed of the foot, the occurrence timing of the movement in the left and right direction and the rotation in the left and right direction of the waist, the strength of stepping on the foot, and the motion amount of the supporting foot.

In addition, the relationship likelihood value calculation unit 406 may calculate the relationship likelihood value using the occurrence time interval of the feature amounts of the motion model, the occurrence order of the feature amounts, a combination of the feature amounts, or the feature amount itself, or any combination thereof.

When a plurality of motion models are used, the adjustment motion section determination unit 407 determines that the adjustment motion section candidate is the adjustment motion section for the main motion section if at least one of the relationship likelihood values calculated for the motion models is a predetermined threshold value or more.

Here, a calculation example of the relationship likelihood value when each of the motion models is used is described. The smooth-normal model may have, as feature amounts, the ratio between the movement amounts of the waist in the front and back direction and the left and right direction, the rotational speed of the foot, and the strength of stepping on the foot. For example, the smooth-normal model has a predetermined threshold value for the feature amount used in the conditions described below. Here, the strength of stepping on the foot may be the strength of stepping on the heel of the foot or the strength of stepping on the entire back of the foot, but in the present embodiment, it is defined as the strength of stepping on the heel of the foot and may be referred to as "heel strike strength".

Then, when the following condition is satisfied, the relationship likelihood value calculation unit 406 calculates the relationship likelihood value of the smooth-normal model higher comparing to that when the following condition is not satisfied. The condition is that the movement amount of the waist in the front and back direction is as large as the movement amount in the left and right direction multiplied by a predetermined threshold value in the adjustment motion section candidate, and/or the rotational speed of the foot and the heel strike strength in the main motion section are within predetermined threshold value ranges. For example, the relationship likelihood value calculation unit 406 increments the likelihood value of the smooth-normal model by 1 when the movement amount of the waist in the front and back direction in the adjustment motion section candidate is as large as the movement amount in the left and right direction multiplied by a predetermined threshold value.

Next, a calculation example of the relationship likelihood value when the smooth-abnormal model is used is described. The smooth-abnormal model may have, as feature amounts, the movement amounts of the waist in the front and back direction and the left and right direction, the rotational speed of the waist in the front and back direction, the rotational speed of the waist in the left and right direction, and the heel strike strength. For example, the smooth-abnormal model has a predetermined threshold value for a feature amount used in the condition described below. Then, when the following condition is satisfied, the relationship likelihood value calculation unit 406 calculates the relationship likelihood value of the smooth-abnormal model higher comparing to that when the following condition is not satisfied. The condition is that the movement amount of the waist in the front and back direction and/or the left and right direction and the rotational speed of the waist in the adjustment motion section candidate are a predetermined threshold value or more and/or the heel strike strength in the main motion section is a predetermined threshold value or more.

Next, a calculation example of the relationship likelihood value when the non-smooth-normal model is used is described. The non-smooth-normal model may have, as feature amounts, the ratio between the movement amount of the waist in the front and back direction and the movement amount of the waist in the left and right direction, the movement amount in the left and right direction and the rotational speed of the waist, the heel strike strength, and the motion amount of the supporting foot. For example, the non-smooth-normal model has a predetermined threshold value for the feature amount used in the conditions described below. Then, when at least one of the following three conditions is satisfied, the relationship likelihood value calculation unit 406 calculates the relation likelihood value of the non-smooth-normal model higher comparing to that when any of the conditions is not satisfied. Here, the relationship likelihood value calculation unit 406 may calculate the relationship likelihood value higher when any one of the three conditions is satisfied, or may calculate the relationship likelihood value higher when all of the three conditions are satisfied. The first condition among the three conditions is that the movement amount of the waist in the left and right direction is as large as the movement amount of the waist in the front and back direction multiplied by the threshold value. The second condition among the three conditions is that there is an order relation that the left and right rotation of the waist occurs within a predetermined threshold time after the occurrence of the movement of the waist in the left and right direction. The third condition among the three conditions is that the heel strike strength and the motion amount of the supporting foot are predetermined threshold values or less.

Next, a calculation example of the relationship likelihood value when the non-smooth-abnormal model is used is described. The non-smooth-abnormal model may have, as feature amounts, the value indicating the synchronization of the motions of the waist and foot, and the length of period during which the waist and the foot move in synchronization. For example, the non-smooth-abnormal model has a predetermined threshold value for the feature amount used in the conditions described below. Then, when at least one of the following two conditions is satisfied, the relationship likelihood value calculation unit 406 calculates the relation likelihood value of the non-smooth-abnormal model higher comparing to that when any of the conditions is not satisfied. Here, the relationship likelihood value calculation unit 406 may calculate the relationship likelihood value higher when either one of the two conditions is satisfied, or may calculate the relationship likelihood value higher when all of the two conditions are satisfied. The first of the two conditions is that the correlation value between the sequential peaks of angular velocity in the front and back direction at the position of the waist and sequential peaks of the angular velocity in the front and back direction at the position of the foot is a predetermined threshold value or more. The second of the two conditions is that the crossing period of the period between the sequential peaks of the angular velocity in the front and back direction at the position of the waist and the period between the sequential peaks of the angular velocity in the front and back direction at the position of the foot is a predetermined threshold value or more.

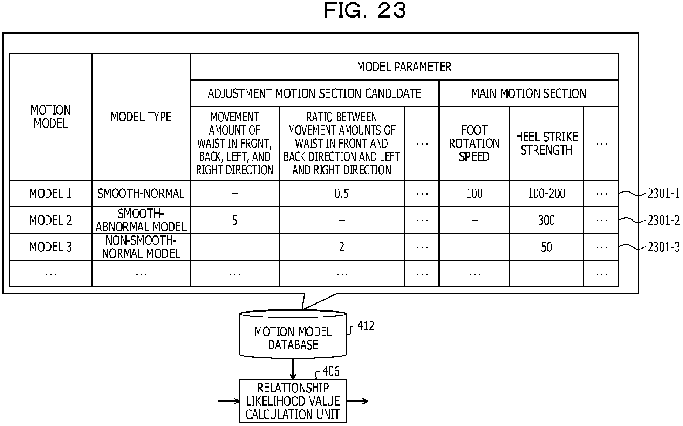

The predetermined threshold values of the four motion models described above are stored in the motion model database 412. A specific example of the motion model database 412 is illustrated in FIG. 23.

In addition, when there is a plurality of adjustment motion section candidates, the output control unit 409 may classify the adjustment motion section candidates into a plurality of groups based on the relationship likelihood value of each adjustment motion section candidate of the plurality of adjustment motion section candidates. Then, the output control unit 409 may output information indicating the classified adjustment motion section candidates corresponding to each group of the plurality of groups. Here, the information indicating an adjustment motion section candidate is, for example, the start time and the end time of the adjustment motion section candidate.

In addition, the adjustment motion section evaluation unit 408 may calculate the evaluation value of the somatosensation based on the relationship likelihood value of each adjustment motion section candidate classified in any group of the plurality of groups and the determination result as to whether the adjustment motion section candidate is the adjustment motion section for the main motion section.

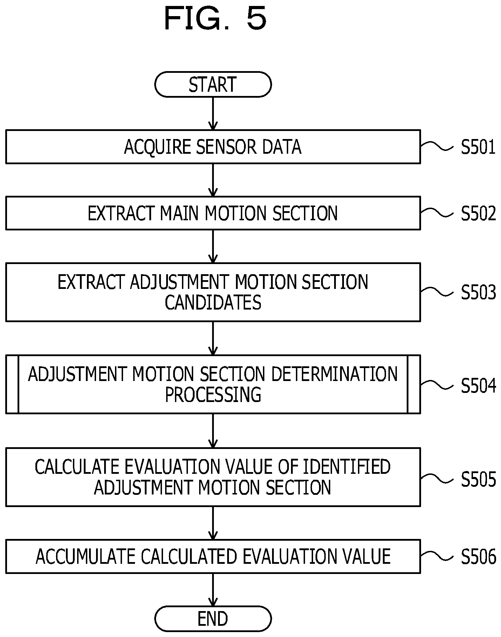

FIG. 5 is a flowchart illustrating an example of the somatosensation evaluation processing procedure. The sensor data acquisition unit 401 acquires sensor data (step S501). Next, the main motion section extraction unit 402 extracts a main motion section (step S502). An example of extraction of the main motion section by the main motion section extraction unit 402 will be described with reference to FIG. 7. Then, the adjustment motion section candidate extraction unit 403 extracts an adjustment motion section candidates (step S503). An example of extraction of the adjustment motion section candidates by the adjustment motion section candidate extraction unit 403 will be described with reference to FIG. 8.

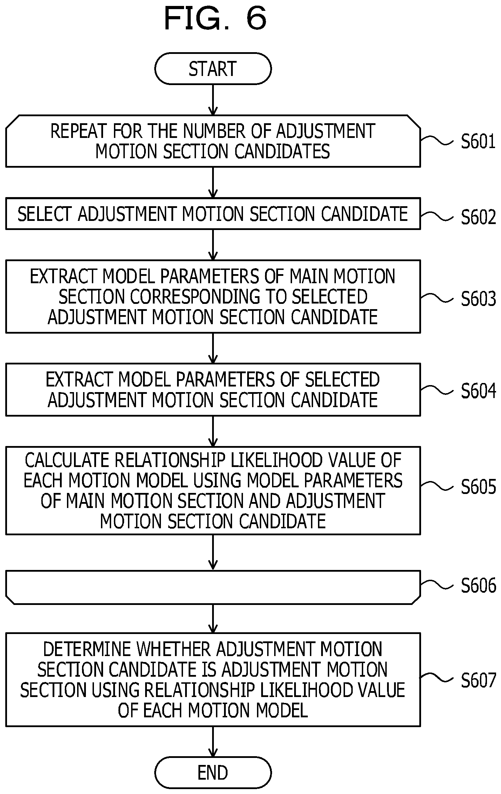

Next, the main motion section parameter extraction unit 404, the adjustment motion section candidate parameter extraction unit 405, the relationship likelihood value calculation unit 406, and the adjustment motion section determination unit 407 cooperate to perform the adjustment motion section determination processing (step S504). The adjustment motion section determination processing will be described with reference to FIG. 6.

Then, the adjustment motion section evaluation unit 408 calculates the evaluation value of the determined adjustment motion section (step S505). Next, the adjustment motion section evaluation unit 408 accumulates the calculated evaluation value in the evaluation value database 413 (step S506). After the processing of step S506 ends, the information processing device 101 ends the somatosensation evaluation processing.

FIG. 6 is a flowchart illustrating an example of the adjustment motion section determination processing procedure. The main motion section parameter extraction unit 404, the adjustment motion section candidate parameter extraction unit 405, and the relationship likelihood value calculation unit 406 repeat the processing of steps S601 to S606 for the number of adjustment motion section candidates. The main motion section parameter extraction unit 404 selects an adjustment motion section candidate (step S602), and extracts model parameters of the main motion section corresponding to the selected adjustment motion section candidate (step S603). An example of extraction of a model parameter of the main motion section by the main motion section parameter extraction unit 404 is described with reference to FIG. 9.

Next, the adjustment motion section candidate parameter extraction unit 405 extracts model parameters of the selected adjustment motion section candidate (step S604). An example of extraction of a model parameter of the adjustment motion section candidate by the adjustment motion section candidate parameter extraction unit 405 is described with reference to FIG. 10. In addition, the adjustment motion section candidate parameter extraction unit 405 may perform the processing of step S603 before the processing of step S602. Further, the main motion section parameter extraction unit 404 and the adjustment motion section candidate parameter extraction unit 405 may perform the processing of step S602 and the processing of step S603 in parallel.

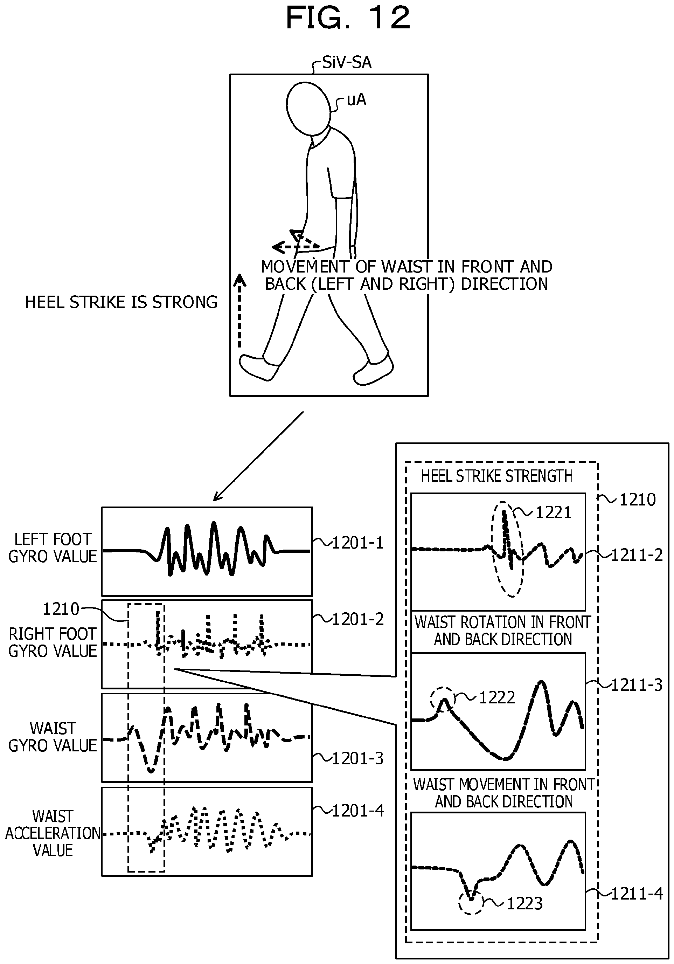

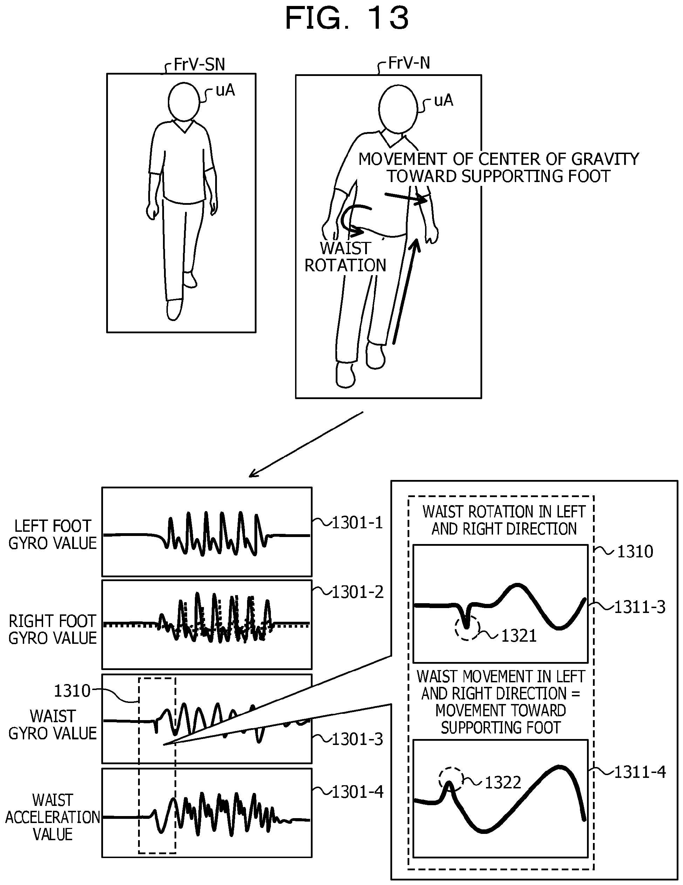

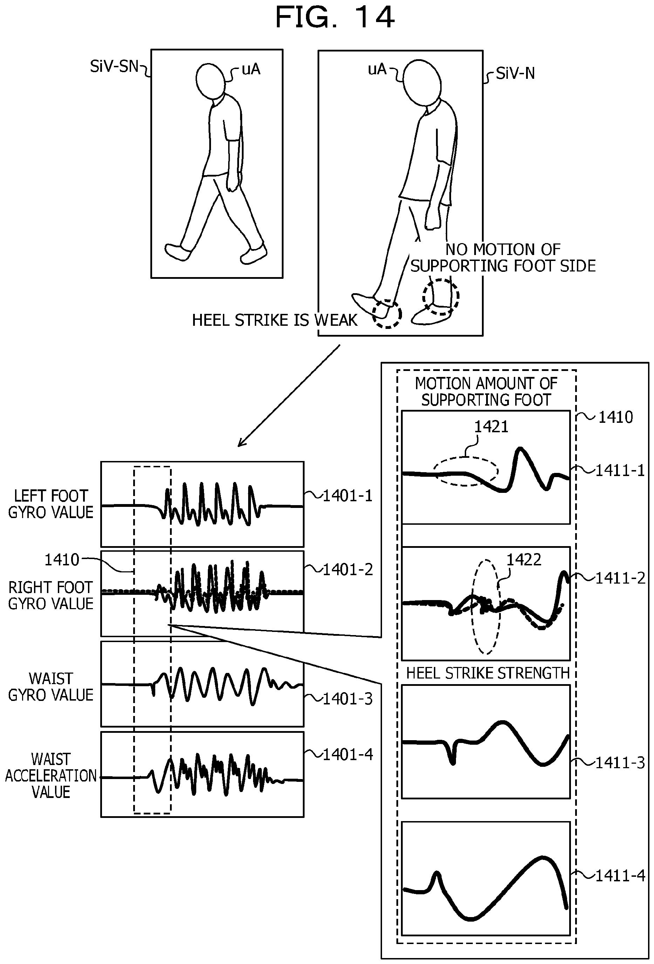

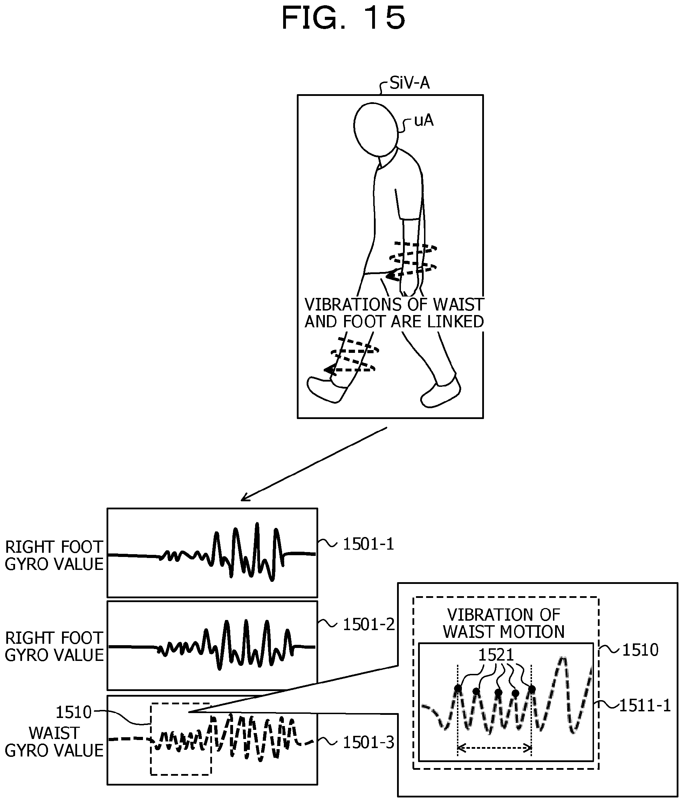

Then, the relationship likelihood value calculation unit 406 calculates the relationship likelihood value of each motion model using model parameters of the main motion section and the adjustment motion section candidate (step S605). Specifically, the relationship likelihood value calculation unit 406 calculates the relationship likelihood value of the smooth-normal model, the relationship likelihood value of the smooth-abnormal model, the relationship likelihood value of the non-smooth-normal model, and the relationship likelihood value of the non-smooth-abnormal model. A calculation example of the relationship likelihood value of the non-smooth-normal model is described with reference to FIG. 11. A calculation example of the relationship likelihood value of the smooth-abnormal model is described with reference to FIG. 12. A calculation example of the relationship likelihood value of the non-smooth-normal model is described with reference to FIGS. 13 and 14. A calculation example of the relationship likelihood value of the non-smooth-abnormal model is described with reference to FIG. 15. The relationship likelihood value calculation unit 406 may calculate the above-described four relationship likelihood values in any order, or may calculate them in parallel.

After repeating the processing of steps S601 to S606 for the number of adjustment motion section candidates, the adjustment motion section determination unit 407 determines whether each of the adjustment motion section candidates is the adjustment motion section using the relationship likelihood value of each motion model (step S607). After the processing of step S607 ends, the information processing device 101 ends the adjustment motion section determination processing.

FIG. 7 is an explanatory diagram illustrating an example of extraction of a main motion section. The main motion section extraction unit 402 may extract the walking motion from the sensor data utilizing, for example, the description of Reference Document 6 stated below. In addition, for example, the main motion section extraction unit 402 may extract the motion of standing up/sitting down from the sensor data utilizing the description of Reference Document 7 stated below.

(Reference Document 6: Paolo Fraccaro and three others, "Real-world Gyroscope-based Gait Event Detection and Gait Feature Extraction", eTELEMED, 2014)

(Reference Document 7: Van Lummel and six others, "Automated approach for quantifying the repeated sit-to-stand using one body fixed sensor in young and older adults", Gait & Posture, Vol. 38, pp 153-156, 2013)

Graphs 701-1 to 701-4 illustrated in FIG. 7 respectively indicate a left foot gyro value, a right foot gyro value, a waist gyro value, and a waist acceleration value among sensor data when a user starts walking from a static state. Each of the graphs 701-1 to 701-4 represents only one axis as a representative. Specifically, the graphs 701-1 and 701-2 each represent the frontal-horizontal axis rotation direction, the graph 701-3 represents the horizontal axis rotation direction, and the graph 701-4 represents the left and right direction.

For example, from the sensor data, the main motion section extraction unit 402 extracts a section mm1 that is a section in which the variance value is a predetermined value or less as a static section. Similarly, from the sensor data, the main motion section extraction unit 402 extracts a section mm2 having a periodic waveform as a walking start section. In FIG. 7, the sections mm1 and mm2 are illustrated as black rectangles. Then, the main motion section extraction unit 402 may extract the sections mm1 and mm2 as main motion sections.

FIG. 8 is an explanatory diagram illustrating an example of extraction of adjustment motion section candidates. The adjustment motion section candidate extraction unit 403 may extract, for example, a transient section itself or a section between transient sections in the description of Japanese Laid-open Patent Publication No. 2016-158887 as an adjustment motion section candidate from the sensor data. Further, the adjustment motion section candidate extraction unit 403 may extract a section that has not been extracted as the main motion sections in the sensor data as an adjustment motion section candidate.

In FIG. 8, the adjustment motion section candidate extraction unit 403 extracts transition sections ts1 and ts2 described in Japanese Laid-open Patent Publication No. 2016-158887 as adjustment motion section candidates from the graphs 701-1 to 701-4. In FIG. 8, the sections ts1 and ts2 are illustrated as shaded rectangles. In addition, graphs 801-1 to 801-4 illustrated in FIG. 8 represent the transient section ts2 extracted as the adjustment motion section candidate from the graphs 701-1 to 701-4.

Further, in FIG. 8, the adjustment motion section candidate ts2 and the main motion section mm2 partially overlap each other. As illustrated in the drawing, the adjustment motion section candidate and the main motion section may partially overlap each other.

FIG. 9 is an explanatory diagram illustrating an example of extraction of model parameters of the main motion section corresponding to each adjustment motion section candidate. The main motion section parameter extraction unit 404 extracts, as model parameters of the main motion section, the feature of the waist movement from the extracted main motion section. The feature of the waist movement is, for example, an amount indicating the rotational speed of a foot, the heel strike strength, the motion amount of the supporting foot, or the vibration of the foot motion. Model parameters 900 of the main motion section illustrated in FIG. 9 represent a portion indicated by the model parameters of the main motion section out of the angular velocity at the position of the waist.

The rotational speed of the foot is, for example, a peak of the angular velocity in the front and back direction at the position of the foot or the like.

The heel strike strength is, for example, an acceleration peak in the gravity direction at the position of the waist, or a peak of acceleration in the front, back, left, and right direction at the position of the foot, specifically the position of the ankle, or a composite value of peaks of acceleration in the front and back direction and the left and right direction, and peaks of angular velocity in the front and back direction and the left and right direction. The heel strike strengths are particularly preferably high frequency peaks extracted by a high pass filter or the like. The graph 901 in the model parameters 900 of the main motion section illustrates angular velocity peaks at a high frequency. The solid line in the graph 901 indicates the frontal-horizontal axis rotational gyro value, and the broken line in the graph 901 indicates the sagittal-horizontal axis rotational gyro value. The angular velocity peak values in frames 911 and 912 in the graph 901 represent the values of the model parameters in the main motion section.

The motion amount of the supporting foot is, for example, an acceleration at a position of one foot in a period extracted as a stepping out period from the angular velocity peaks at the position of the other foot, or a variance value of the acceleration values. Graphs 902 and 903 in the model parameters 900 of the main motion section represent the angular velocities at the positions of the other foot and the one foot, respectively. Then, the variance value of the angular velocity in a frame 913 in the stepping out period that can be extracted from the graph 903 is the value of the model parameter of the main motion section.

The amount indicating the vibration of the foot motion is, for example, sequential front and back rotational angular velocity peak values at the position of the foot or the like. In addition, the model parameter may be, for example, speed, distance, time, or the like during the motion.

The model parameter extracted from the main motion section may be changed depending on the type of motion section, such as a static section or a walking start section, but it is preferable to extract all of the model parameters illustrated in FIG. 9.

FIG. 10 is an explanatory diagram illustrating an example of extraction of model parameters of each adjustment motion section candidate. The adjustment motion section candidate parameter extraction unit 405 extracts, as model parameters of the adjustment motion section, the features of the movement of the waist, and the trunk such as the center of gravity from the extracted adjustment motion section. The feature of the trunk movement is, for example, the movement amount of the waist in the front, back, left, and right direction, the ratio between the movement amounts of the waist in the front and back direction and the left and right direction, the rotational speed of the waist in the front, back, left, and right direction, the occurrence timings of the movement of the waist in the left and right direction and the rotation of the waist in the left and right direction, and an amount indicating the vibration of the waist motion. A model parameter 1000 of an adjustment motion section candidate illustrated in FIG. 10 represents a graph 1001 of sensor data of an acceleration at the position of the waist and a graph 1002 of sensor data of an angular velocity at the position of the waist.

The movement amount of the waist in the front, back, left, and right direction is, for example, a peak of acceleration or an integral value thereof at the position of the waist in the front, back, left, and right direction, or the like. Here, in the graph 1001 of sensor data of acceleration at the position of the waist, a solid line indicates an acceleration value in the left and right direction, and a broken line indicates an acceleration value in the front and back direction. Then, for example, acceleration peak values at points 1011 and 1012 in the sensor data graph 1001 are values of model parameters of the adjustment motion section candidate.

The ratio of the movement amount of the waist in the front, back, left, and right direction is, for example, a ratio between acceleration peak in the front and back direction and acceleration peak in the left and right direction or integral values thereof at the position of the waist, or the like. For example, the ratio between acceleration peaks at points 1011 and 1012 is a value of the model parameters of the adjustment motion section candidate.

The rotational speed of the waist in the front, back, left, and right direction is, for example, a peak of angular velocity at the position of the waist in the front, back, left, and right direction, or the like.

The occurrence timings of the movement in the left and right direction and the rotation in the left and right direction of the waist are, for example, the time of acceleration peak in the left and right direction and the time of angular velocity peak in the left and right direction at the position of the waist, or the like.

The amount indicating the vibration of the waist motion is, for example, the magnitude of small peaks or the length of period of sequential small peaks of the front and back rotational angular velocity at the position of the waist or the like. The small peak is a local minimum value or a local maximum value having a difference from the average smaller than a certain value. Here, the broken line in the graph 1002 of the sensor data of the angular velocity at the position of the waist indicates the frontal-horizontal axis rotational gyro value. Points 1021-1 to 1021-5 in the graph 1002 of sensor data are sequential small peaks indicating vibration. Thus, the length of period from the time of the point 1021-1 to the time of the point 1021-5 is a model parameter of the adjustment motion section candidate.

FIG. 11 is an explanatory diagram illustrating a calculation example of the relationship likelihood value of the smooth-normal model. The relationship likelihood value calculation unit 406 calculates the relationship likelihood value of the smooth-normal model higher if the adjustment motion section candidate has a feature of an adjustment motion for moving the waist in the front and back direction by a certain distance or longer at a certain speed or higher at the start of walking, and a motion of making the first step at a certain speed or higher after the adjustment motion, for example.

The upper part of FIG. 11 presents an image FrV-SN of the user uA at the start of walking viewed from the front and an image SiV-SN of the user uA from the side when the user uA is a healthy person. The lower left part of FIG. 11 illustrates sensor data pieces 1101-1 to 1101-3 as an example of the motions of the waist and foot when the user uA starts walking. The sensor data pieces 1101-1 to 1101-3 respectively indicate the left foot gyro value in the frontal-horizontal axis rotation direction, the right foot gyro value in the frontal-horizontal axis rotation direction, and the waist acceleration value in the front and back direction. A frame 1110 in the sensor data pieces 1101-2 and 1101-3 indicate that the section is an adjustment motion section candidate. The lower right portion of FIG. 11 illustrates a sensor data piece 1111-2 in the frame 1110 in the sensor data piece 1101-2 and a sensor data piece 1111-3 in a frame 1110 in the sensor data piece 1101-3.

For example, the relationship likelihood value calculation unit 406 uses the first peak value of the right foot gyro value and the acceleration peak value of the waist in the front and back direction illustrated in frames 1121 and 1122 in the sensor data pieces 1111-2 and 1111-3 to calculate the relationship likelihood value of the smooth-normal model.

FIG. 12 is an explanatory diagram illustrating a calculation example of the relationship likelihood value of the smooth-abnormal model. The relationship likelihood value calculation unit 406 calculates the relationship likelihood value of the smooth-abnormal model higher if the adjustment motion section candidate has a feature of an adjustment motion for moving the waist in the front and back direction by a certain distance or longer at a certain speed or higher at the start of walking, and then a heal strike of a certain strength or more, for example.

The upper part of FIG. 12 illustrates an image SiV-SA of the user uA viewed from the side when the user uA loses the balance and makes a false step. As illustrated in the image SiV-SA, when the user uA loses the balance and makes a false step, there is a feature that the waist moves in the front and back (left and light) walk, and the heal strike is strong.

The lower left part of FIG. 12 illustrates sensor data pieces 1201-1 to 1201-4 as an example of the motions of the waist and foot when the user uA makes a false step. The sensor data pieces 1201-1 to 1201-4 respectively indicate the left foot gyro value in the frontal-horizontal axis rotation direction, the right foot gyro value in the sagittal-horizontal axis rotation direction, the waist gyro value in the frontal-horizontal axis rotation direction, and the waist acceleration value in the front and back direction. A frame 1210 in the sensor data pieces 1201-2 and 1201-4 indicates that the section is an adjustment motion section candidate. In addition, the lower right part of FIG. 12 illustrates sensor data pieces 1211-2 to 1211-4 in the frame 1210 in the sensor data pieces 1201-2 to 1201-4.

For example, the relationship likelihood value calculation unit 406 may calculate the first peak value of the right foot gyro value, the first peak value of the waist gyro value, and the peak value of the waist acceleration value indicated by frames 1221 to 1223 in the sensor data pieces 1211-2 to 1211-4 themselves as the relationship likelihood values of the smooth-abnormal model.

FIG. 13 is an explanatory diagram (No. 1) illustrating a calculation example of the relationship likelihood value of the non-smooth-normal model. The relationship likelihood value calculation unit 406 calculates the relationship likelihood value of the non-smooth-normal model higher if the adjustment motion section candidate has a feature of a motion for moving the waist toward the supporting foot by a certain distance or longer at the start of walking, for example.

The upper part of FIG. 13 presents, for comparison, an image FrV-SN of the user uA who has started walking viewed from the front when the user uA is a healthy person, and an image FrV-N of the user uA who has started walking viewed from the front when the user uA is a concussion patient. As illustrated in the image FrV-N, when the user uA who is a concussion patient starts walking, there is a feature that a rotation of the waist of the user uA occurs and the center of gravity moves toward the supporting foot.

The lower left part of FIG. 13 illustrates sensor data pieces 1301-1 to 1301-4 as an example of the motions of the waist and foot when the user uA who is a concussion patient starts walking. The sensor data pieces 1301-1 to 1301-4 respectively indicate the left foot gyro value in the frontal-horizontal axis rotation direction, the right foot gyro values in the frontal-horizontal axis and sagittal-horizontal axis rotation directions, the waist gyro value in the frontal-horizontal axis rotation direction, and the waist acceleration value in the front and back direction. A frame 1310 in the sensor data pieces 1301-3 and 1301-4 indicates that the section is an adjustment motion section candidate. The lower right portion of FIG. 13 illustrates a sensor data piece 1311-3 in the frame 1310 in the sensor data piece 1301-3 and a sensor data piece 1311-4 in a frame 1310 in the sensor data piece 1301-4.

For example, the relationship likelihood value calculation unit 406 uses the first peak value of the waist gyro value and the first peak value of the acceleration value of the waist illustrated in frames 1321 and 1322 in the sensor data pieces 1311-3 and 1311-4 to calculate the relationship likelihood value of the non-smooth-normal model.

FIG. 14 is an explanatory diagram (No. 2) illustrating a calculation example of the relationship likelihood value of the non-smooth-normal model. In the non-smooth-normal model, the main motion is a normal motion, and is, for example, stepping out slowly and steadily or the like as a result of a small movement amount of the waist in the front and back direction at the time of adjustment motion making a propulsive force of the first step weak. For example, the non-smooth-normal model is a stable heel strike at the first step, that is, a weak heel strike strength, no motion of the supporting foot side linked with the stepping-out foot due to the waist not moving forward, or the like. Therefore, the relationship likelihood value calculation unit 406 may calculate the relationship likelihood value of the non-smooth-normal model higher if the adjustment motion section candidate has a feature of performing the motion described above.

The upper part of FIG. 14 presents, for comparison, an image SiV-SN of the user uA who has started walking viewed from the side when the user uA is a healthy person, and an image SiV-N of the user uA who has started walking viewed from the side when the user uA is a concussion patient. As illustrated in the image SiV-N, when the user uA who is a concussion patient starts walking, there is a feature that the heal strike is weak and there is no motion of the supporting foot side.

The lower left part of FIG. 14 illustrates sensor data pieces 1401-1 to 1401-4 as an example of the motions of the waist and foot when the user uA who is a concussion patient starts walking. The sensor data pieces 1401-1 to 1401-4 respectively indicate the left foot gyro value in the frontal-horizontal axis rotation direction, the right foot gyro values in the frontal-horizontal axis and sagittal-horizontal axis rotation directions, the waist gyro value in the vertical axis rotation direction, and the waist acceleration value in the left and right direction. A frame 1410 in the sensor data pieces 1401-1 and 1401-4 indicates that the section is an adjustment motion section candidate. In addition, the lower right part of FIG. 14 illustrates sensor data pieces 1411-1 to 1411-4 in the frame 1410 in the sensor data pieces 1401-1 to 1401-4.

For example, the relationship likelihood value calculation unit 406 uses the variation value of the left foot gyro value and the peak value of the right foot gyro value in a certain section as the motion amounts of the supporting foot illustrated in the frames 1421 and 1422 in the sensor data pieces 1411-1 and 1411-2 to calculate the relationship likelihood value of the non-smooth-normal model.

In addition, the relationship likelihood value calculation unit 406 may perform either one of the method illustrated in FIG. 13 and the method illustrated in FIG. 14 or both of them for calculating the relationship likelihood value of the non-smooth-normal model.

FIG. 15 is an explanatory diagram illustrating a calculation example of the relationship likelihood value of the non-smooth-abnormal model. The relationship likelihood value calculation unit 406 calculates the relationship likelihood value of the non-smooth-abnormal model higher if the adjustment motion section candidate has a feature of the adjustment motion in which the center of gravity vibrates and the main motion of making short steps after the adjustment motion, for example.

The upper part of FIG. 15 presents an image SiV-A of the user uA making Freezing of Gait or walking with short steps viewed from the side when the user uA is a patient of a cranial nervous system disease. As illustrated in the image SiV-A, when the user uA who is a patient of a cranial nervous system disease makes Freezing of Gait and walks with short steps, there is a feature that the vibrations of the waist and the foot link.

The lower left part of FIG. 15 illustrates sensor data pieces 1501-1 to 1501-3 as an example of the motions of the waist and the foot when the user uA who is a patient of a cranial nervous system disease makes Freezing of Gait and walks with short steps. The sensor data pieces 1501-1 to 1501-3 respectively indicate the left foot gyro value in the frontal-horizontal axis rotation direction, the right foot gyro value in the sagittal-horizontal axis, and the waist gyro value in the frontal-horizontal axis rotation direction. A frame 1510 in the sensor data piece 1501-3 indicates that the section is an adjustment motion section candidate. In addition, the lower right part of FIG. 15 illustrates a sensor data piece 1511-1 in the frame 1510 in the sensor data piece 1501-3.

For example, the relationship likelihood value calculation unit 406 uses the length of time period between sequential peaks within a certain threshold value as the vibration of the motion of the waist illustrated as points 1521 in the sensor data piece 1511-1 to calculate the relationship likelihood value of the non-smooth-abnormal model.

(Calculation Example of Relationship likelihood value of Each Motion Model)

In addition, the relationship likelihood value calculation unit 406 may calculate the relationship likelihood value using the occurrence time interval of the feature amounts of the motion model, the occurrence order of the feature amounts, a combination of the feature amounts, or the feature amount itself, or any combination thereof as illustrated in FIG. 4.

(Example of Determination of Adjustment Motion Section)

The adjustment motion section determination unit 407 may determine, for example, an adjustment motion section candidate itself having the relationship likelihood value of a particular motion model of a certain threshold value or more, or a section before or after the adjustment motion section having the relationship likelihood value of a certain threshold value or more as the adjustment motion section.

(Calculation Example of Evaluation Value)

The adjustment motion section evaluation unit 408 categorizes the sections determined as the adjustment motion sections using the motion model and the relationship likelihood value by the adjustment motion section determination unit 407, and performs comparison and evaluation. As an evaluation, the adjustment motion section evaluation unit 408 may calculate an evaluation value based on the relationship likelihood value of each motion model with respect to the adjustment motion section. For example, the adjustment motion section evaluation unit 408 may calculate, as the evaluation value representing abnormality of the somatosensation, the sum of the values each obtained by multiplying the relationship likelihood value of one of the motion model by a weight value using the following expression (1).

$$L = \alpha_{SN}M_{SN}(x|\theta_{SN}) + \alpha_{SA}M_{SA}(x|\theta_{SA}) + \alpha_{N}M_{N}(x|\theta_{N}) + \alpha_{A}M_{A}(x|\theta_{A}) \quad \ldots \quad (1)$$

In the expression, L represents an evaluation value. $\alpha_{SN}$, $\alpha_{SA}$, $\alpha_{N}$, and $\alpha_{A}$ are weight values of the smooth-normal model, the smooth-abnormal model, the non-smooth-normal model, and the non-smooth-abnormal model, respectively. $\alpha_{SN}$, $\alpha_{SA}$, $\alpha_{N}$, and $\alpha_{A}$ satisfy the magnitude relation represented by the following expression (2).

$$\alpha_{SN} < \alpha_{SA} < \alpha_{N} < \alpha_{A} \quad \ldots \quad (2)$$

x indicates sensor data in the adjustment motion section. $\theta_{SN}$, $\theta_{SA}$, $\theta_{N}$, and $\theta_{A}$ are parameters of the smooth-normal model, the smooth-abnormal model, the non-smooth-normal model, and the non-smooth-abnormal model, respectively. $M_{SN}(x|\theta_{SN})$, $M_{SA}(x|\theta_{SA})$, $M_{N}(x|\theta_{N})$, $M_{A}(x|\theta_{A})$ are the relationship likelihood values of the smooth-normal model, the smooth-abnormal model, the non-smooth-normal model, and the non-smooth-abnormal model, respectively.

Further, the adjustment motion section evaluation unit 408 may calculate the evaluation value by comparing the relationship likelihood value or the feature amount of the determined adjustment motion section with the relationship likelihood value or the feature amount that is a reference. Here, as an example of determination of the relationship likelihood value and the feature amount serving as the reference, the adjustment motion section evaluation unit 408 first extracts a motion model having the highest relationship likelihood value with respect to each of the adjustment motion section, and then classifies sections for which the same motion model is extracted into the same category. Then, the adjustment motion section evaluation unit 408 may determine the relationship likelihood value or the feature amount of any one section of the classified sections, or the average relationship likelihood value or feature amount of the section of a certain range, as the relationship likelihood value or the feature amount as a reference.

(Specific Example of First Embodiment)

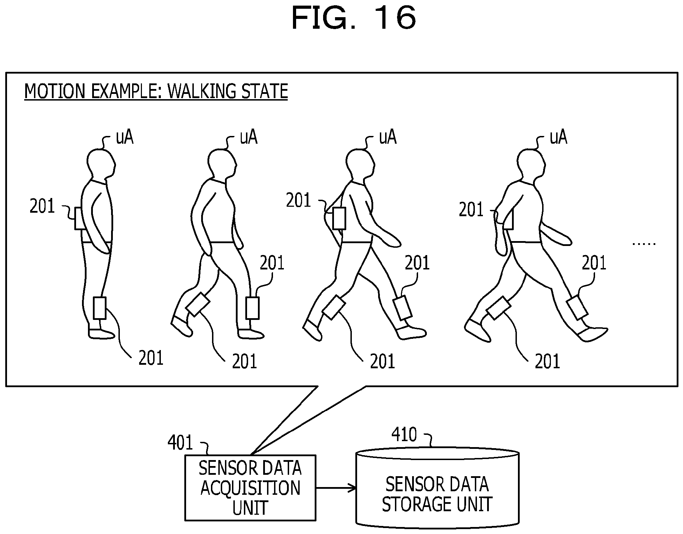

Next, as a specific example of the first embodiment, an example in which the motion of a patient uA who is a concussion patient is measured during testing or during daily life, and based on the measured motion, a posture adjustment section is detected and somatosensation is evaluated is described with reference to FIGS. 16 to 27.

FIG. 16 is an explanatory diagram illustrating an example of a walking state of the patient uA in the first embodiment. In the first embodiment, it is assumed that the data acquisition device 201 is attached to the waist of the user uA and then attached to each of the left and right feet. FIG. 15 illustrates the patient uA walking during testing and daily life. The sensor data acquisition unit 401 of the data acquisition device 201 attached to each portion of the patient uA measures the motion of the portion, and stores the measured sensor waveform in the sensor data storage unit 410.

FIG. 17 is an explanatory diagram illustrating an example of sensor data during walking of the patient uA in the first embodiment. FIG. 17 illustrates a gyro value of each of the portions. Specifically, the sensor data pieces 1701-1 to 1701-4 indicate sensor data pieces of the left foot gyro value, the right foot gyro value, the waist gyro value, and the waist acceleration value respectively at each time. Here, the sensor 102 of the data acquisition device 201 measures the acceleration and the gyro value in the directions of three axes. However, in FIG. 17, in order to simplify the illustration, the gyro value is illustrated only in the frontal-horizontal axis rotation direction, and the acceleration is illustrated only in the up and down direction. The same applies to the following description unless otherwise specified. The information processing device 101 acquires sensor data of each portion from the sensor data storage unit 410.

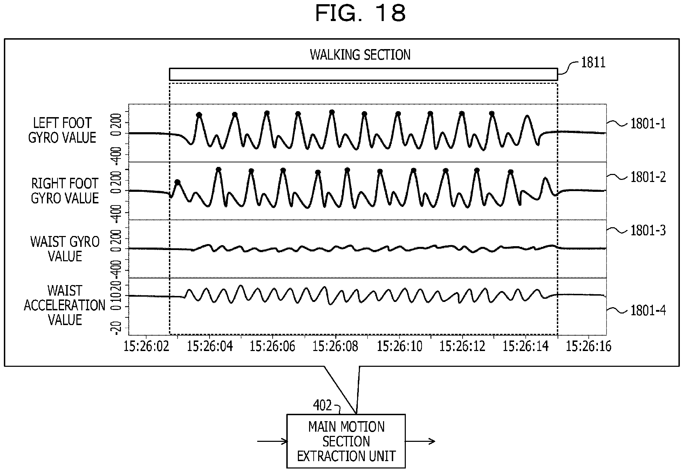

FIG. 18 is an explanatory diagram illustrating an example of extraction of a main motion section in the first embodiment. The main motion section extraction unit 402 extracts the main motion section from the sensor data by likelihood value determination based on the motion models, using the methods described in Reference Document 6 and Reference Document 7.

FIG. 18 illustrates an example in which a walking section is determined as the main motion section using sensor data pieces 1801-1 to 1801-4. The walking section has features including the peak values of left and right feet gyro values, intervals between peaks of the left and right feet gyro values, and the numbers of peaks of left and right feet gyro values. The peak values of left and right feet gyro values are gyro values indicated by black circles of the sensor data pieces 1801-1 and 1801-2. The intervals between peaks of the left and right feet gyro values are periods between the black circles in the sensor data pieces 1801-1 and 1801-2. The peak numbers of peaks of left and right feet gyro values are the numbers of black circles in the sensor data pieces 1801-1 and 1801-2.

The main motion section extraction unit 402 increments the likelihood value score when a certain threshold range of each feature of the walking section is satisfied. Then, the main motion section extraction unit 402 extracts a section as the walking section when the total value obtained by the incrementation is a threshold value or more. In the example of FIG. 18, the main motion section extraction unit 402 extracts a section 1811 as the walking section. In FIG. 18, the walking section is illustrated as a white rectangle. The same applies to the following drawings.

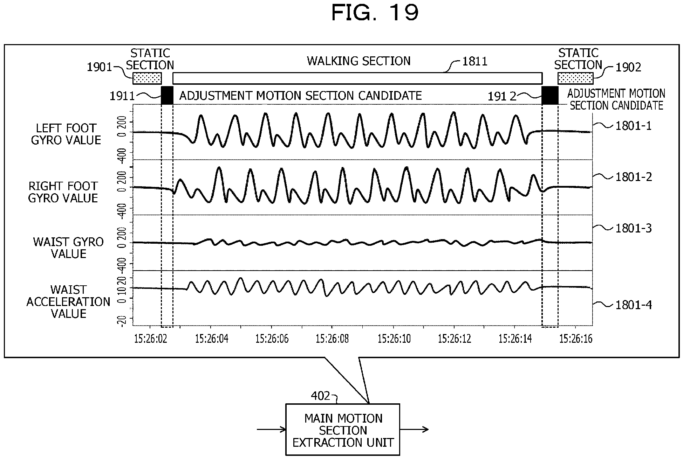

FIG. 19 is an explanatory diagram (No. 1) illustrating an example of extraction of an adjustment motion section candidate in the first embodiment. The adjustment motion section candidate extraction unit 403 extracts an adjustment motion section candidate from the sensor data using the method described with reference to FIG. 8.

In the example of FIG. 19, it is assumed that the main motion section extraction unit 402 extracts the static section 1901, the walking section 1811, and the static section 1902 in order of time from the sensor data pieces 1801-1 to 1801-4. In the example of FIG. 19, the static section is illustrated as a shaded rectangle. The same applies to the following drawings.

In this case, the adjustment motion section candidate extraction unit 403 extracts adjustment motion section candidates 1911 and 1912 in order of time from the sensor data pieces 1801-1 to 1801-4. In the example of FIG. 19, the adjustment motion section candidates are illustrated as black rectangles. The same applies to the following drawings.

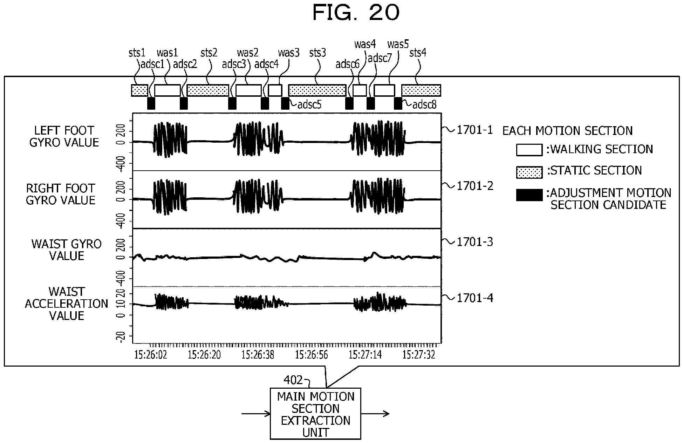

FIG. 20 is an explanatory diagram (No. 2) illustrating an example of extraction of an adjustment motion section candidate in the first embodiment. FIG. 20 illustrates an example, in which, from the sensor data pieces 1701-1 to 1701-4 illustrated in FIG. 17, the main motion section extraction unit 402 extracts walking sections and static sections as main motion sections, and the adjustment motion section candidate extraction unit 403 extracts posture adjustment section candidates.

The extracted sections are a static section sts1, a posture adjustment section candidate adsc1, a walking section was1, a posture adjustment section candidate adsc2, a static section sts2, a posture adjustment section candidate adsc3, a walking section was2, a posture adjustment section candidate adsc4 in order of time. Furthermore, after the posture adjustment section candidate adsc4, a walking section was3, a posture adjustment section candidate adsc5, a static section sts3, a posture adjustment section candidate adsc6, a walking section was4, a posture adjustment section candidate adsc7, a walking section was5, and a posture adjustment section candidate adsc8 follow. In addition, after the posture adjustment section candidate adsc8, a static section sts4 follows.

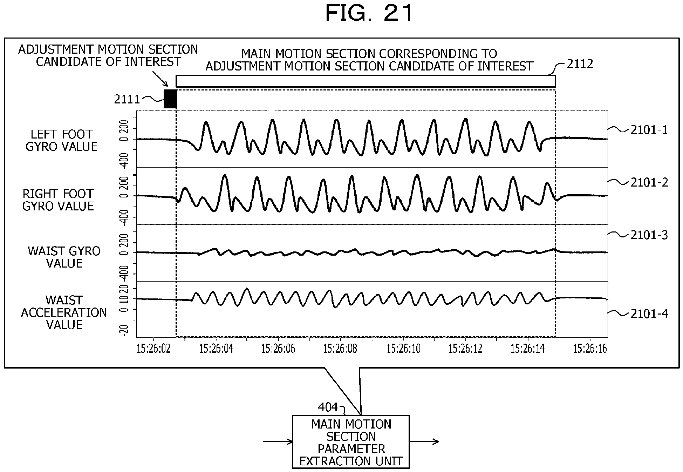

FIG. 21 is an explanatory diagram illustrating an example of extraction of model parameters of the main motion section corresponding to each adjustment motion section candidate in the first embodiment. The main motion section parameter extraction unit 404 extracts model parameters of the main motion section corresponding to each adjustment motion section using the method described with reference to FIG. 9.

FIG. 21 illustrates an example in which model parameters of the main motion section are extracted using sensor data pieces 2101-1 to 2101-4. In FIG. 21, the main motion section parameter extraction unit 404 extracts model parameters of a main motion section 2112 corresponding to an adjustment motion section candidate 2111 of interest.

For example, the main motion section parameter extraction unit 404 extracts the feature amounts of the main motion section described below as model parameters. The feature amounts are, for example, the rotational speed of a foot, the heel strike strength, the motion amount of the supporting foot, or the vibration of the foot motion. The rotational speed of the foot is a peak of the rotational angular velocity in the frontal-horizontal axis of the foot. The heel strike strength is a high frequency peak value extracted from the angular velocity of a foot by a high pass filter. The motion amount of the supporting foot is a variance value of acceleration velocities at a position of one foot in a period extracted as a stepping out period from the angular velocity peaks at the position of the other foot. The vibration of the foot motion is sequential front and back rotational angular velocity peak values of the foot.

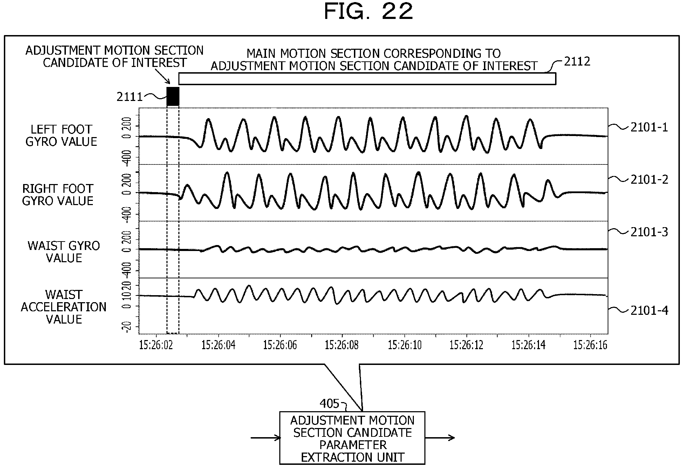

FIG. 22 is an explanatory diagram illustrating an example of extraction of model parameters of an adjustment motion section candidate in the first embodiment. The adjustment motion section candidate parameter extraction unit 405 extracts model parameters of the adjustment motion section candidate using the method described with reference to FIG. 10.

FIG. 22 illustrates an example in which model parameters of the adjustment motion section candidate are extracted using sensor data pieces 2101-1 to 2101-4. In FIG. 22, the adjustment motion section candidate parameter extraction unit 405 extracts model parameters of the adjustment motion section candidate 2111 of interest.

For example, the adjustment motion section candidate parameter extraction unit 405 extracts the feature amounts of the adjustment motion section candidate described below as model parameters. The feature amount is, for example, the movement amount of the waist in the front, back, left, and right direction, the ratio between the movement amounts of the waist in the front and back direction and the left and right direction, the rotational speed of the waist in the front, back, left, and right direction, the occurrence timings of the movement of the waist in the left and right direction and the rotation of the waist in the left and right direction, and an amount indicating the vibration of the waist motion. The movement amount of the waist in the front, back, left, and right direction is an acceleration peak value at the position of the waist in the front, back, left, and right direction. The ratio of the movement amount of the waist in the front, back, left, and right direction is a ratio between acceleration peak values in the front and back direction and the left and right direction at the position of the waist. The rotational speed of the waist in the front, back, left, and right direction is a peak value of the angular velocity at the position of the waist in the front, back, left, and right direction. The occurrence timings of the movement in the left and right direction and the rotation in the left and right direction of the waist are the time of acceleration peak value in the left and right direction and the time of angular velocity peak value in the left and right direction at the position of the waist. The amount indicating the vibration of the waist motion is the magnitude of small peaks of sequential small peaks of the front and back rotational angular velocity at the position of the waist.

FIG. 23 is an explanatory diagram (No. 1) illustrating a calculation example of the relationship likelihood value of each motion model according to the first embodiment. The motion model database 412 illustrated in FIG. 23 has records 2301-1 to 2301-3.

The motion model database 412 includes fields of a motion model, a model type, and a model parameter. In the motion model field, a character string identifying a motion model is stored. In the model type field, a character string indicating a motion model type is stored. In the model parameter field, values of model parameters of an adjustment motion section candidate and a main motion section are stored.

The record 2301-1 indicates the smooth-normal model, the record 2301-2 indicates the smooth-abnormal model, and the record 2301-3 indicates the non-smooth-normal model.

The values of the model parameters in the motion model database 412 may be set by a vendor providing a program according to the present embodiment, or may be set by a doctor using the program according to the present embodiment.

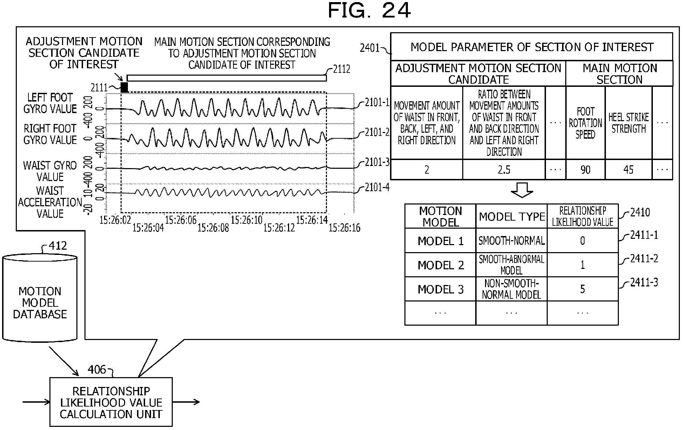

FIG. 24 is an explanatory diagram (No. 2) illustrating a calculation example of the relationship likelihood value of each motion model according to the first embodiment. The relationship likelihood value calculation unit 406 calculates the relationship likelihood value of each motion model with reference to the motion model database 412. For example, when the model parameters of the adjustment motion section candidate 2111 and the main motion section 2112 of interest are similar to the model parameters of a motion model in the motion model database 412, the relationship likelihood value calculation unit 406 increments the relationship likelihood value of the motion model.

A table 2401 illustrated in FIG. 24 is a list of model parameters of the sections of interest, that is, the adjustment motion section candidate 2111 and the main motion section 2112. When each of the model parameters listed in the table 2401 is similar to a model parameter of a motion model in the motion model database 412, the relationship likelihood value calculation unit 406 increments the relationship likelihood value of the motion model.

In the example of FIG. 24, the relationship likelihood value calculation unit 406 compares the motion model database 412 and the table 2401 illustrated in FIG. 24, and generates a list 2410 of relationship likelihood values of the adjustment motion section candidates 2111. The list 2410 illustrated in FIG. 24 has records 2411-1 to 2411-3.

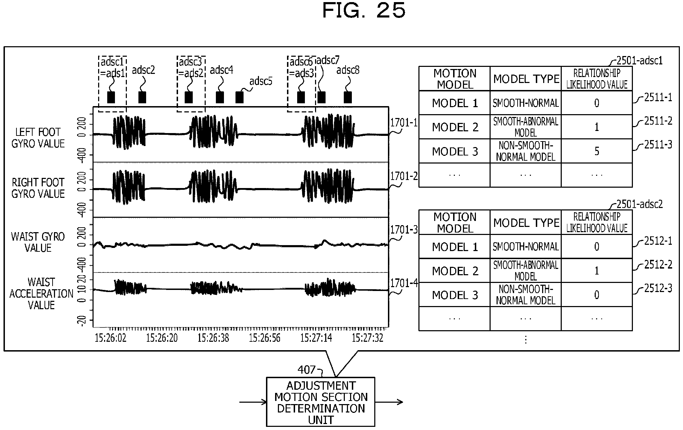

FIG. 25 is an explanatory diagram illustrating an example of determination of an adjustment motion section in the first embodiment. In FIG. 25, an example in which the adjustment motion section determination unit 407 determines whether each of the adjustment motion section candidates adsc1 to adsc8 extracted from the sensor data 1701-1 to 1701-4 illustrated in FIG. 17 is an adjustment motion section.

As illustrated in FIG. 25, the relationship likelihood value calculation unit 406 calculates the relationship likelihood value for each of the adjustment motion section candidates adsc1 to adsc8. A list 2501-adsc1 illustrated in FIG. 25 illustrates the relationship likelihood values of the adjustment motion section candidate adsc1, and a list 2501-adsc2 illustrates the relationship likelihood values of the adjustment motion section candidate adsc2.

In the example of FIG. 25, the adjustment motion section determination unit 407 then determines that an adjustment motion section candidate having a relationship likelihood value of a certain value or more is the adjustment motion section. In FIG. 25, the adjustment motion section determination unit 407 determines the adjustment motion section candidates adsc1, adsc3 and adsc6 surrounded by a broken line frame as the adjustment motion sections ads1, ads2 and ads3.

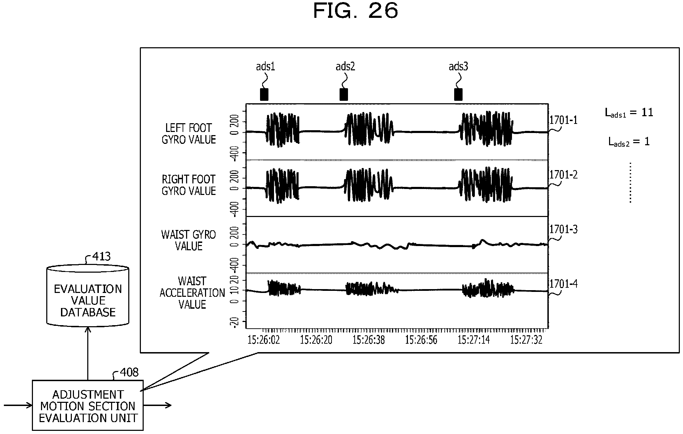

FIG. 26 is an explanatory diagram illustrating an example of calculation of an evaluation value in the first embodiment. The adjustment motion section evaluation unit 408 calculates the evaluation value of the determined adjustment motion section. In FIG. 26, the adjustment motion section evaluation unit 408 calculates the evaluation value of the adjustment motion section, for example, according to expression (1). $L_{ads1}$ illustrated in FIG. 26 is the calculated evaluation value of the adjustment motion section ads1, and $L_{ads2}$ is the calculated evaluation value of the adjustment motion section ads2.

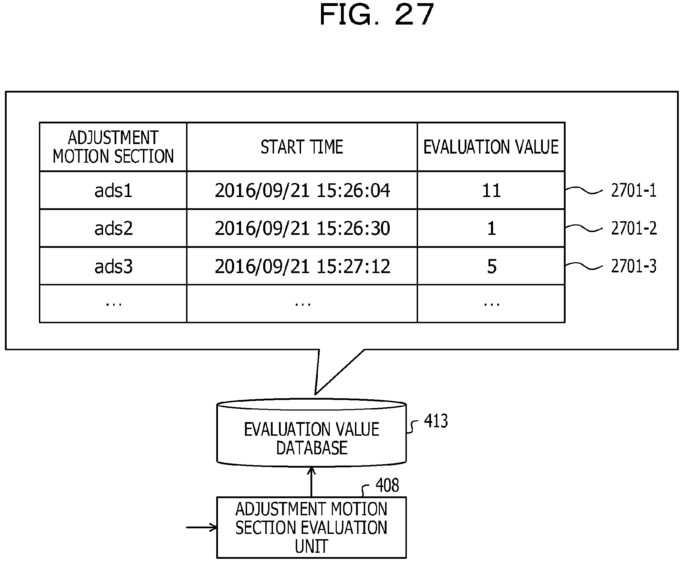

FIG. 27 is an explanatory diagram illustrating an example of accumulation of evaluation values in the first embodiment. The adjustment motion section evaluation unit 408 accumulates calculated evaluation values in the evaluation value database 413. The evaluation value database 413 illustrated in FIG. 27 has records 2701-1 to 2701-3. The records 2701-1 to 2701-3 are records of the adjustment motion sections ads1 to ads3, respectively.

As described above, the information processing device 101 calculates the relationship likelihood value based on the feature amounts of the main motion section and the adjustment motion section candidate extracted from the sensor data of the patient, and determines whether the above-described adjustment motion section candidate is an adjustment motion section based on the calculated relationship likelihood value. Thereby, the information processing device 101 may improve the determination accuracy as to whether the adjustment motion section candidate is an adjustment motion section since the information processing device 101 uses the relationship between motions in two sections of the main motion section and the adjustment motion section candidate.

Further, the information processing device 101 may calculate the evaluation value of the somatosensation of the patient based on the determination result as to whether the adjustment motion section candidate is an adjustment movement section and the calculated relationship likelihood value. Thereby, the information processing device 101 may provide a doctor or other persons with the evaluation value of the somatosensation of the patient. Then, the doctor may, for example, continuously review the evaluation value of the somatic sense of the patient to detect a decrease in the somatosensation or the like of the patient.

Further, when there is a plurality of adjustment motion section candidates, the information processing device 101 may classify each adjustment motion section candidate of the plurality of adjustment motion section candidates into any of a plurality of groups based on the relationship likelihood value, and output the classified adjustment motion section candidates corresponding to each group to the group. Thereby, the information processing device 101 may, for example, group similar scenes by grouping the adjustment motion section candidates based on the relationship likelihood values. For example, the information processing device 101 may classify the adjustment motion section candidates into a group of walking on a soft ground and a group of walking on a hard ground by classifying based on the relationship likelihood values. Further, the plurality of adjustment motion section candidates may be adjustment motion section candidates of a plurality of patients. In this case, the information processing device 101 may classify the adjustment motion section candidates into groups each for a symptom based on the relationship likelihood.

In addition, when there is a plurality of adjustment motion section candidates, the information processing device 101 may calculate the evaluation value of the somatosensation of the patient using the adjustment motion section candidates classified into any one of the plurality of groups. Thereby, the information processing device 101 may provide, for example, the evaluation value of only the classified adjustment motion section candidates to a doctor.

Further, the information processing device 101 may calculate the relationship likelihood value of the adjustment motion section candidate using a motion model representing a combination of the features of the main motion and the adjustment motion. Thereby, the information processing device 101 can calculate the relationship likelihood value taking the combination of the features of the main motion and the adjustment motion into consideration.

A motion model may be a model having a model parameter related to the movement of the waist or a model parameter related to the movement of the foot to be described below, or any combination thereof. The model parameters related to the motion of the waist are, for example, a movement amount of the waist in the front and back direction and/or the left and right direction, the ratio between the movement amount of the waist in the front and back direction and the movement amount of the waist in the left and right direction, the rotational speed of the waist in the front and back direction and/or the left and right direction, or an amount indicating the vibration of the motion of the waist. The model parameters related to the movement of the foot are the rotational speed of the foot, the occurrence timing of the movement in the left and right direction and the rotation in the left and right direction of the waist, the strength of stepping on the foot, and the motion amount of the supporting foot. Thereby, the information processing device 101 may extract the feature of the main motion accompanied by the movement such as walking, turning, stair climbing, or the change of posture, or the feature of the adjustment motion during transition from the main motion to another main motion.

In addition, the information processing device 101 may calculate the relationship likelihood value based on the occurrence time interval of the feature amounts of the motion model, the occurrence order of the feature amounts, a combination of the feature amounts, or the feature amount, or any combination thereof. The information processing device 101 may reduce the amount of data to be held by not holding the feature amount that is not used.

The information processing device 101 may determine that the adjustment motion section candidate is the adjustment motion section if at least one of the relationship likelihood values calculated for each motion model of the plurality of motion models is a predetermined threshold value or more. As a result, the information processing device 101 may determine that the adjustment motion section candidate is the adjustment motion section when the adjustment motion section candidate corresponds to any of the plurality of combinations of the features of the main motion and the adjustment motion, thereby improving the determination accuracy.

Further, the information processing device 101 may use the smooth-normal model as one motion model of the plurality of motion models. Thereby, the information processing device 101 may determine that the adjustment motion section candidate is the adjustment motion section when a motion that appears on a healthy person is found, thereby improving the determination accuracy.

Further, the information processing device 101 may use the smooth-abnormal model as one motion model of the plurality of motion models. Thereby, the information processing device 101 may determine that the adjustment motion section candidate is the adjustment motion section when a motion of a false step caused by losing the balance is found, thereby improving the determination accuracy.

Further, the information processing device 101 may use the non-smooth-normal model as one motion model of the plurality of motion models. Thereby, the information processing device 101 may determine that the adjustment motion section candidate is the adjustment motion section when a motion that appears on a concussion patient is found, thereby improving the determination accuracy.

Further, the information processing device 101 may use the non-smooth-abnormal model as one motion model of the plurality of motion models. Thereby, the information processing device 101 may determine that the adjustment motion section candidate is the adjustment motion section when a motion that appears on a patient of a cranial nervous system disease such as Parkinson's disease is found, thereby improving the determination accuracy.

The information processing method described in the present embodiment can be implemented by executing a prepared program on a computer such as a personal computer or a workstation. The present information processing program is recorded on a computer-readable recording medium such as a hard disk, flexible disk, CD-ROM(Compact Disc-Read Only Memory) or DVD(Digital Versatile Disk), and is read from the recording medium to be executed by the computer. Moreover, the present information processing program may be distributed via a network such as the Internet.

REFERENCE SIGNS LIST

    100 information processing system
    101 information processing device
    102 sensor
    111 main motion section
    112 adjustment motion section candidate
    311 control unit
    400 sensor data acquisition unit
    401 sensor data acquisition unit
    402 main motion section extraction unit
    403 adjustment motion section candidate extraction unit
    404 main motion section parameter extraction unit
    405 adjustment motion section candidate parameter extraction unit
    406 relationship likelihood value calculation unit
    407 adjustment motion section determination unit
    408 adjustment motion section evaluation unit
    409 output control unit
    410 sensor data storage unit
    412 motion model database
    413 evaluation value database

**Claims**

1. An information processing system comprising: a sensor configured to measure at least a motion among a state or the motion of a living body or an object; and an information processing device capable of communicating with the sensor, wherein

    the information processing device is configured to
    extract, from time-series data obtained by the sensor, a first data section, in which the state of the living body or the object is changed to a predetermined state or the living body or the object makes a predetermined motion,
    extract, from the time-series data, a second data section different from the first data section,
    calculate, based on a feature amount of the first data section and a feature amount of the second data section, a likelihood value that indicates likelihood that the second data section is an adjustment motion section during which a motion for adjusting a posture of the living body or the object is made for the first data section, and
    determine, based on the likelihood value that is calculated, whether the second data section is an adjustment motion section for the first data section.

2. The information processing system according to claim 1, wherein the information processing device is further configured to calculate an evaluation value for evaluating somatosensation of the living body or the object based on a determination result as to whether the second data section is an adjustment motion section for the first data section and a feature amount representing the adjustment motion section.

3. The information processing system according to claim 1 or 2, wherein the information processing device is further configured to

when the second data section is provided in a plurality, classify each of the second data sections into one of a plurality of groups based on a likelihood value corresponding to the second data section, and
output information indicating the classified adjustment motion section candidates corresponding to each group to the group.

4. The information processing system according to claim 3, wherein the information processing device is further configured to calculate an evaluation value for evaluating somatosensation of the living body or the object based on the likelihood value of the second data section that is classified into one group among the plurality of groups and a determination result as to whether the second data section is an adjustment motion section for the first data section.

5. The information processing system according to any one of claims 1 to 4, wherein the calculating processing calculates the likelihood value that the second data section is an adjustment motion section for the first data section based on motion models, the feature amount of the first data section, and the feature amount of the second data section, the motion models indicating combinations of whether the predetermined state or the predetermined motion of the living body or the object is normal and whether the motion for adjusting the predetermined state or the predetermined motion is smooth.

6. The information processing system according to claim 5, wherein

the living body is a patient, and
the motion models are models each having a feature amount that is an amount, a speed, or an occurrence timing of movement and/or rotation of a waist and/or a foot of the living body in a left and right direction and/or a front and back direction, or any combination thereof, and/or a motion amount, a strength of stepping, or a vibration of the waist and/or the foot of the living body, or any combination thereof.

7. The information processing system according to claim 5, wherein the calculating processing calculates the likelihood value that the second data section is an adjustment motion section for the first data section based on an occurrence time interval of the feature amounts of each of the motion models, an occurrence order of the feature amounts, a combination of the feature amounts, or the feature amounts, or any combination thereof.

8. The information processing system according to any one of claims 1 to 7, wherein

the calculating calculates the likelihood value that the second data section is an adjustment motion section for the first data section based on each of the motion models, the feature amount of the first data section, and the feature amount of the second data section corresponding to the motion model of the plurality of motion models that are different from each other in whether the predetermined state or the predetermined motion of the living body or the object is normal, whether the motion for adjusting the predetermined state or the predetermined motion is smooth, the feature amount of the predetermined state or the predetermined motion or the adjustment motion, or the value of the feature amount of the predetermined state or the predetermined motion or the adjustment motion, or any combination thereof, and
the determining processing determines that the second data section is an adjustment motion section for the first data section when at least one of the likelihood values calculated corresponding to the motion models is a predetermined threshold value or more.

9. The information processing system according to claim 8, wherein

the living body is a patient,
one motion model of the plurality of motion models has a ratio between a movement amount of the waist of the living body in the front and back direction and a movement amount of the waist of the living body in the left and right direction, a rotational speed of the foot of the living body, and a strength of stepping on the foot as the feature amounts, and
in the one motion model, the calculating processing calculates the likelihood value higher when a condition is satisfied comparing to the likelihood value when the condition is not satisfied, the condition being that the movement amount of the waist in the front and back direction in the second data section is as large as the movement amount of the waist in the left and right direction multiplied by a predetermined threshold value, and/or the rotational speed of the foot in the first data section and the strength of stepping on the foot in the first data section is within a predetermined threshold value range.

10. The information processing system according to claim 8 or 9, wherein

the living body is a patient,
one motion model of the plurality of motion models has a movement amount of the waist of the living body in the front and back direction and a movement amount of the waist of the living body in the left and right direction, a rotational speed of the waist in the front and back direction, a rotation speed of the waist in the left and right direction, and the strength of stepping on the foot of the living body as the feature amounts, and
in the one motion model, the calculating processing calculates the likelihood value higher when a condition is satisfied comparing to the likelihood value when the condition is not satisfied, the condition being that the movement amount in the front and back direction and/or the movement amount in the left and right direction of the waist, and the rotation speed of the waist in the second data section is a predetermined threshold value or more, and/ or the strength of stepping on the foot in the first data section is a predetermined threshold value or more.

11. The information processing system according to any one of claims 8 to 10, wherein

the living body is a patient,
one motion model of the plurality of motion models has a ratio between a movement amount of the waist of the living body in the front and back direction and a movement amount of the waist of the living body in the left and right direction, the movement amount of the waist in the left and right direction, a rotational speed of the waist in the left and right direction, a strength of stepping on the foot of the living body, and a motion amount of a supporting foot as feature amounts, and
in the one motion model, the calculating processing calculates the likelihood value higher when at least one of conditions is satisfied comparing to the likelihood value when any of the condition is not satisfied, the conditions including a condition that the movement amount of the waist in the left and right direction in the second data section is as large as the movement amount of the waist in the front and back direction multiplied by a predetermined threshold value, a condition that the rotation of the waist in the left and right direction occurs within a predetermined threshold time after occurrence of the movement of the waist in the left and right direction in the second data section, and a condition that the strength of stepping on the foot and the motion amount of the supporting foot in the first data section are predetermined threshold values or less.

12. The information processing system according one of claims 8 to 11, wherein

the living body is a patient,
one motion model of the plurality of motion models has a value indicating synchronization of motions of the waist of the living body and the foot of the living body, and a length of period during which the waist and the foot move in synchronization as feature amounts, and
in the one motion model, the calculating processing calculates the likelihood value higher when a condition is satisfied comparing to the likelihood value when the condition is not satisfied, the condition being that a correlation value between sequential peak values of angular velocity in the front and back direction at a position of the waist in the second data section and sequential peak values of rotational angular velocity in the front and back direction at a position of the foot in the first data section is a predetermined threshold value or more, and/or a period during which a period between the sequential peaks of the rotational angular velocity in the front and back direction at the position of the waist in the second data section and the period between the sequential peaks of the rotational angular velocity in the front and back direction at the position of the foot in the first data section cross is a predetermined threshold value or more.

13. An information processing device capable of communicating with a sensor configured to measure at least a motion among a state or the motion of a living body or an object, comprising

a control unit configured to
extract, from time-series data obtained by the sensor, a first data section, in which the state of the living body or the object is changed to a predetermined state or the living body or the object makes a predetermined motion,
extract, from the time-series data, a second data section different from the first data section,
calculate, based on a feature amount of the first data section and a feature amount of the second data section, a likelihood value that indicates likelihood that the second data section is an adjustment motion section during which a motion for adjusting a posture of the living body or the object is made for the first data section, and determine, based on the likelihood value that is calculated, whether the second data section is an adjustment motion section for the first data section.

**14.** An information processing method performed by an information processing device capable of communicating with a sensor configured to measure at least a motion among a state or the motion of a living body or an object, comprising

extracting, from time-series data obtained by the sensor, a first data section, in which the state of the living body or the object is changed to a predetermined state or the living body or the object makes a predetermined motion; extracting, from the time-series data, a second data section different from the first data section; calculating, based on a feature amount of the first data section and a feature amount of the second data section, a likelihood value that indicates likelihood that the second data section is an adjustment motion section during which a motion for adjusting a posture of the living body or the object is made for the first data section; and determining, based on the likelihood value that is calculated, whether the second data section is an adjustment motion section for the first data section.

# FIG. 1

100

uA      uA      uA

102      102      102

(1) ACQUIRE

101

INFORMATION PROCESSING DEVICE

(3) EXTRACT                                    (2) EXTRACT

ADJUSTMENT MOTION                ADJUSTMENT MOTION
SECTION CANDIDATE                SECTION CANDIDATE

112                    111

103

113 113 113 113 113 113 113 113 113

114

LARGE MOVEMENT AMOUNT OF WAIST        PERIODIC MOVEMENT OF WAIST
IN FRONT AND BACK DIRECTION          IN FRONT AND BACK DIRECTION

(4) CALCULATE
RELATIONSHIP LIKELIHOOD VALUE: HIGH

(5) DETERMINE

DETERMINE THAT ADJUSTMENT MOTION SECTION
CANDIDATE IS ADJUSTMENT MOTION SECTION

# FIG. 2

# FIG. 3

INFORMATION PROCESSING DEVICE 101

301 CONTROL UNIT
309
302 MAIN MEMORY
303 AUXILIARY STORAGE UNIT
304 DRIVE DEVICE
306 NETWORK I/F UNIT
307 INPUT UNIT
308 OUTPUT UNIT
305 STORAGE MEDIUM

210
202 HUB

DATA ACQUISITION DEVICE 201

102 SENSOR
315
311 CONTROL UNIT
314 NETWORK I/F UNIT
MAIN MEMORY
312
313 AUXILIARY STORAGE UNIT

# FIG. 4

201
DATA ACQUISITION DEVICE

401
SENSOR DATA
ACQUISITION
UNIT

410
SENSOR DATA
STORAGE UNIT

100

101
INFORMATION PROCESSING DEVICE

411 STORAGE UNIT

412 MOTION MODEL
DATABASE

413 EVALUATION
VALUE DATABASE

301 CONTROL UNIT

402
MAIN
MOTION
SECTION
EXTRACTION
UNIT

403
ADJUSTMENT
MOTION
SECTION
CANDIDATE
EXTRACTION
UNIT

404
MAIN
MOTION
SECTION
PARAMETER
EXTRACTION
UNIT

405
ADJUSTMENT
MOTION
SECTION
CANDIDATE
PARAMETER
EXTRACTION
UNIT

406
RELATIONSHIP
LIKELIHOOD
VALUE
CALCULATION
UNIT

407
ADJUSTMENT
MOTION
SECTION
DETERMINATION
UNIT

408
ADJUSTMENT
MOTION
SECTION
EVALUATION
UNIT

409
OUTPUT
CONTROL
UNIT

# FIG. 5

START

ACQUIRE SENSOR DATA — S501

EXTRACT MAIN MOTION SECTION — S502

EXTRACT ADJUSTMENT MOTION SECTION CANDIDATES — S503

ADJUSTMENT MOTION SECTION DETERMINATION PROCESSING — S504

CALCULATE EVALUATION VALUE OF IDENTIFIED ADJUSTMENT MOTION SECTION — S505

ACCUMULATE CALCULATED EVALUATION VALUE — S506

END

# FIG. 6

START

REPEAT FOR THE NUMBER OF ADJUSTMENT MOTION SECTION CANDIDATES — S601

SELECT ADJUSTMENT MOTION SECTION CANDIDATE — S602

EXTRACT MODEL PARAMETERS OF MAIN MOTION SECTION CORRESPONDING TO SELECTED ADJUSTMENT MOTION SECTION CANDIDATE — S603

EXTRACT MODEL PARAMETERS OF SELECTED ADJUSTMENT MOTION SECTION CANDIDATE — S604

CALCULATE RELATIONSHIP LIKELIHOOD VALUE OF EACH MOTION MODEL USING MODEL PARAMETERS OF MAIN MOTION SECTION AND ADJUSTMENT MOTION SECTION CANDIDATE — S605

— S606

DETERMINE WHETHER ADJUSTMENT MOTION SECTION CANDIDATE IS ADJUSTMENT MOTION SECTION USING RELATIONSHIP LIKELIHOOD VALUE OF EACH MOTION MODEL — S607

END

# FIG. 7

mm1

mm2

LEFT FOOT GYRO VALUE

400
200
0
-200
-400

701-1

RIGHT FOOT GYRO VALUE

400
200
0
-200
-400

701-2

WAIST GYRO VALUE

50
0
-50

701-3

WAIST ACCELERATION VALUE

5
4
3
2
1
0

701-4

09:11:23    09:11:27    09:11:31    09:11:35
09:11:25    09:11:29    09:11:33

# FIG. 8

# FIG. 9

900

MODEL PARAMETERS OF MAIN MOTION SECTION

SENSOR WAVEFORMS OF ANGULAR VELOCITIES AT POSITIONS OF FEET

VARIANCE VALUE OF ACCELERATION VELOCITIES

ANGULAR VELOCITY PEAK

911
901 913
912
902

ONE FOOT

903

—— FRONTAL-HORIZONTAL AXIS ROTATIONAL GYRO VALUE

----- SAGITTAL-HORIZONTAL AXIS ROTATIONAL GYRO VALUE

STEPPING OUT PERIOD

THE OTHER FOOT

# FIG. 10

# FIG. 11

# FIG. 12

SiV-SA

uA

MOVEMENT OF WAIST IN FRONT AND BACK (LEFT AND RIGHT) DIRECTION

HEEL STRIKE IS STRONG

LEFT FOOT GYRO VALUE — 1201-1

1210 — RIGHT FOOT GYRO VALUE — 1201-2

WAIST GYRO VALUE — 1201-3

WAIST ACCELERATION VALUE — 1201-4

HEEL STRIKE STRENGTH — 1210

1221 — 1211-2

WAIST ROTATION IN FRONT AND BACK DIRECTION

1222 — 1211-3

WAIST MOVEMENT IN FRONT AND BACK DIRECTION

1223 — 1211-4

# FIG. 13

# FIG. 14

SiV-SN — uA

uA — SiV-N

NO MOTION OF SUPPORTING FOOT SIDE

HEEL STRIKE IS WEAK

MOTION AMOUNT OF SUPPORTING FOOT

1421

~1410

~1411-1

1422

~1411-2

HEEL STRIKE STRENGTH

~1411-3

~1411-4

LEFT FOOT GYRO VALUE — ~1401-1

1410 —

RIGHT FOOT GYRO VALUE — ~1401-2

WAIST GYRO VALUE — ~1401-3

WAIST ACCELERATION VALUE — ~1401-4

# FIG. 15

SiV-A

uA

VIBRATIONS OF WAIST AND FOOT ARE LINKED

RIGHT FOOT GYRO VALUE — 1501-1

RIGHT FOOT GYRO VALUE — 1501-2

1510 — WAIST GYRO VALUE — 1501-3

VIBRATION OF WAIST MOTION

1521

1510

1511-1

# FIG. 16

MOTION EXAMPLE: WALKING STATE

uA          uA          uA          uA

201         201         201         201

201         201         201         201

201         201         201         201

.....

401 ⌐

| SENSOR DATA |
| ACQUISITION |
| UNIT |

410 ⌐

SENSOR DATA
STORAGE UNIT

# FIG. 17

# FIG. 18

WALKING SECTION

1811

LEFT FOOT
GYRO VALUE

RIGHT FOOT
GYRO VALUE

WAIST GYRO
VALUE

WAIST
ACCELERATION
VALUE

1801-1

1801-2

1801-3

1801-4

15:26:02  15:26:04  15:26:06  15:26:08  15:26:10  15:26:12  15:26:14  15:26:16

402

MAIN MOTION
SECTION
EXTRACTION UNIT

# FIG. 19

# FIG. 20

FIG. 21

FIG. 22

## FIG. 23

| MOTION MODEL | MODEL TYPE | MODEL PARAMETER | | | | | |
|---|---|---|---|---|---|---|---|
| | | ADJUSTMENT MOTION SECTION CANDIDATE | | | MAIN MOTION SECTION | | |
| | | MOVEMENT AMOUNT OF WAIST IN FRONT, BACK, LEFT, AND RIGHT DIRECTION | RATIO BETWEEN MOVEMENT AMOUNTS OF WAIST IN FRONT AND BACK DIRECTION AND LEFT AND RIGHT DIRECTION | ... | FOOT ROTATION SPEED | HEEL STRIKE STRENGTH | ... |
| MODEL 1 | SMOOTH-NORMAL | − | 0.5 | ... | 100 | 100-200 | ... | 2301-1 |
| MODEL 2 | SMOOTH-ABNORMAL MODEL | 5 | − | ... | − | 300 | ... | 2301-2 |
| MODEL 3 | NON-SMOOTH-NORMAL MODEL | − | 2 | ... | − | 50 | ... | 2301-3 |
| ... | ... | ... | ... | ... | ... | | ... |

MOTION MODEL DATABASE ⌐412

RELATIONSHIP LIKELIHOOD VALUE CALCULATION UNIT ⌐406

## FIG. 24

FIG. 25

# FIG. 26

# FIG. 27

| ADJUSTMENT MOTION SECTION | START TIME | EVALUATION VALUE | |
|---|---|---|---|
| ads1 | 2016/09/21 15:26:04 | 11 | 2701-1 |
| ads2 | 2016/09/21 15:26:30 | 1 | 2701-2 |
| ads3 | 2016/09/21 15:27:12 | 5 | 2701-3 |
| ... | ... | ... | |

EVALUATION VALUE DATABASE 413

ADJUSTMENT MOTION SECTION EVALUATION UNIT 408

## INTERNATIONAL SEARCH REPORT

| | International application No. |
|---|---|
| | PCT/JP2017/012494 |

**A.  CLASSIFICATION OF SUBJECT MATTER**
*A61B5/11*(2006.01)i

According to International Patent Classification (IPC) or to both national classification and IPC

**B.  FIELDS SEARCHED**

Minimum documentation searched (classification system followed by classification symbols)
A61B5/11

Documentation searched other than minimum documentation to the extent that such documents are included in the fields searched
Jitsuyo Shinan Koho        1922–1996   Jitsuyo Shinan Toroku Koho   1996–2017
Kokai Jitsuyo Shinan Koho   1971–2017   Toroku Jitsuyo Shinan Koho   1994–2017

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
|---|---|---|
| A | JP 2016-158887 A  (Fujitsu Ltd.), 05 September 2016 (05.09.2016), entire text; all drawings & US 2016/0256078 A1 entire text; all drawings & EP 3064133 A1 | 1-14 |
| A | JP 2002-78697 A  (Microstone Corp.), 19 March 2002 (19.03.2002), entire text; all drawings (Family: none) | 1-14 |
| A | US 2015/0196231 A1  (Purdue Research Foundation), 16 July 2015 (16.07.2015), entire text; all drawings (Family: none) | 1-14 |

☒  Further documents are listed in the continuation of Box C.      ☐  See patent family annex.

| | |
|---|---|
| *     Special categories of cited documents: | "T"   later document published after the international filing date or priority date and not in conflict with the application but cited to understand the principle or theory underlying the invention |
| "A"   document defining the general state of the art which is not considered    to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 12 May 2017 (12.05.17) | 30 May 2017 (30.05.17) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

**INTERNATIONAL SEARCH REPORT**

| International application No. |
| --- |
| PCT/JP2017/012494 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | Tadashi YAMASHITA et al., "Study of human gait initiation. Regression analysis of experimental data and simulation", Transactions of the Society of Instrument and Control Engineers, vol.22, no.3, 31 March 1986 (31.03.1986), pages 303 to 309 | 1-14 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002078697 A
- JP 2010273746 A
- JP 10024026 A
- JP 2016158887 A

### Non-patent literature cited in the description

- **MARTINA MANCINI.** ANTICIPATORY POSTURAL ADJUSTMENTS PRIOR TO STEP INITIATION ARE HYPOMETRIC IN UNTREATED PARKINSON'S DISEASE: AN ACCELEROMETER-BASED APPROACH. *European Journal of Neurology,* 2009, vol. 16, 1028-1034
- **JEBB G. REMELIUS.** Gait Initiation in Multiple Sclerosis. *Motor Control,* 2008, vol. 12, 93-108
- **RIGOBERTO MARTINEZ-MENDEZ.** Detection of anticipatory postural adjustments prior to gait initiation using inertial wearable sensors. *Journal of NeuroEngineering and Rehabilitation,* 2011, vol. 8 (17
- **MARTINA MANCINI.** Validity and reliability of an IMU-based method to detect APAs prior to gait initiation. *Gait & Posture,* 2016, vol. 43, 125-131
- **DOMEN NOVAK.** Automated detection of gait initiation and termination using wearable sensors. *Medical Engineering & Physics,* 2013, vol. 25, 1713-1720
- **PAOLO FRACCARO.** Real-world Gyroscope-based Gait Event Detection and Gait Feature Extraction. *eTELEMED,* 2014
- **VAN LUMMEL.** Automated approach for quantifying the repeated sit-to-stand using one body fixed sensor in young and older adults. *Gait & Posture,* 2013, vol. 38, 153-156